# EUROPÄISCHE PATENTSCHRIFT

(11) **EP 4 159 257 B1**
(45) Veröffentlichungstag und Bekanntmachung des Hinweises auf die Patenterteilung: **05.08.2026**
(21) Anmeldenummer: 22188664.1
(22) Anmeldetag: 11.05.2007
(51) Int. Cl.: A61M 5/20, A61M 5/24, A61M 5/315

(54) **INJEKTIONSGERÄT MIT VERBESSERTEM FÖRDERELEMENT**
INJECTION DEVICE WITH IMPROVED CONVEYING ELEMENT
APPAREIL D'INJECTION POURVU D'ÉLÉMENT DE TRANSPORT AMÉLIORÉ

(30) Priorität: 15.09.2006 CH 14752006
(43) Veröffentlichungstag der Anmeldung: 05.04.2023
(62) Teilanmeldung aus: 20177292.8
(73) Patentinhaber: Ypsomed AG, 3401 Burgdorf (CH)
(72) Erfinder: Wittmann, Jürgen, 3400 Burgdorf (CH); Kohlbrenner, Philippe, 3413 Kaltacker (CH); Hostettler, Patrick, 3415 Hasle (CH); Stettler, Peter, 3423 Ersigen (CH); Wittwer, Martin, verstorben (CH)
(74) Vertreter: Meier Obertüfer, Jürg

(56) Entgegenhaltungen:
- EP-A1- 1 681 070
- WO-A1-2006/086983

## Beschreibung

### Technisches Gebiet

Die vorliegende Erfindung betrifft eine Vorrichtung zur Verabreichung eines fluiden Produkts. Die Vorrichtung kann als Injektionsgerät zur Injektion einer einstellbaren Dosis des Produkts ausgestaltet sein und insbesondere die Form eines Injektionspens, d.h. eines kompakten Injektionsgeräts in Stiftform, annehmen.

### Stand der Technik

Aus dem Stand der Technik sind eine grosse Zahl von Injektionsgeräten zur dosierten Verabreichung von Medikamenten wie Insulin, Wachstumshormonen oder Osteoporosepräparaten bekannt, welche regelmässig verabreicht werden müssen. Derartige Geräte sollen einerseits zuverlässig und präzise eine voreinstellbare Dosis ausschütten. Andererseits sollen sie in hohem Masse bedienungssicher und benutzerfreundlich sein. Dies gilt umso mehr, da sie in der Regel vom Patienten selbst bedient werden.

Das Medikament kann in einer auswechselbaren Karpule untergebracht sein, die in einen Karpulenhalter einsetzbar ist. Dieser lässt sich dann an einem Gehäuse des Injektionsgeräts befestigen, z.B. durch eine Schraubverbindung oder eine Bajonettverbindung. Zur Ausschüttung wird ein Produktstopfen in der Karpule durch eine Fördereinrichtung mit einem Förderelement in Form einer Kolbenstange vorgeschoben.

Insbesondere sind kompakte, stiftförmige Verabreichungsgeräte bekannt, bei denen die Ausschüttung nach einem ersten Auslösen automatisch erfolgt ("Power-assisted pens"). Die Dosis wird bei solchen Geräten in der Regel durch eine Drehung an einem Dosierknopf voreingestellt. Im Gerät ist ein Antrieb vorhanden, z.B. ein Federantrieb, der beim Einstellen der Dosis gespannt wird. Das Gerät wird durch das Eindrücken einer Auslöseeinrichtung, der mit dem Dosierknopf identisch sein kann, ausgelöst. Dabei erzeugt der Antrieb eine Antriebsbewegung, z.B. in Form einer Drehbewegung, die in eine Vortriebsbewegung der Kolbenstange in eine distale Richtung umgewandelt wird. Im Falle eines Antriebs durch eine Drehbewegung ist die Kolbenstange meist als Gewindestange ausgebildet, auf der eine Antriebsmutter läuft.

In der DE-A 10 2004 063 644, DE-A 10 2004 063 647, WO-A 2004/002556 und DE-A 102 29 122 sind derartige Injektionspens offenbart, die allgemeinen Stand der Technik für die vorliegende Erfindung darstellen.

In der EP-A 1 681 070 ist in der Figuren 7A und 7B ein manuell angetriebener Injektionspen offenbart, dessen Kolbenstange 30 hohl ausgebildet ist und statt eines Aussengewindes ein Innengewinde aufweist. Dieses Innengewinde steht mit dem Aussengewinde einer drehbaren Antriebswelle 31 in Eingriff, die im Bereich ihres proximalen Endes gegenüber einem inneren Gehäuseteil 13 gelagert ist. Die Kolbenstange weist an ihrer Aussenseite durchgehende Längsnuten auf, in die Vorsprünge einer Fixierbuchse 32 eingreifen. Diese Fixierbuchse ist relativ zum Gehäuse festgelegt, wenn ein Behältnishalter 1 mit dem Gehäuse verbunden ist, und kann frei drehen, wenn der Behältnishalter vom Gehäuse gelöst ist. Bei der Verabreichung einer Dosis dreht die Antriebswelle und schiebt dadurch die über die Fixierbuchse drehfest geführte Kolbenstange linear vor. Bei einem Reservoirwechsel wird der Behältnishalter vom Gehäuse gelöst. Dadurch wird die Fixierbuchse und mit ihr auch die Kolbenstange gegenüber dem Gehäuse frei drehbar. Wenn die Kolbenstange nun wieder in das Gehäuse eingeschoben wird, bleibt die Antriebswelle ortsfest, und die Kolbenstange vollführt eine Schraubenbewegung.

Diese Vorrichtung ist in mehrfacher Hinsicht verbesserungsfähig. So erfordert die Schraubenbewegung der Kolbenstange beim Einschieben zwingend, dass an ihrem distalen Ende eine Kappe drehbar angebracht ist, welche beim Einschieben nicht mit der Kolbenstange mitdreht, da sonst beim Einschieben keine geeignete drehfeste Gegenfläche zur Verfügung stünde, an der die zum Einschieben benötigte axiale Kraft angreifen könnte. Des weiteren erfordert die Anwesenheit der Fixierbuchse, dass an der Aussenseite der Kolbenstange Längsnuten ausgebildet sind. In diesen kann sich Schmutz ansammeln. Ausserdem ist eine Lagerung der Antriebswelle relativ zum Gehäuse in ihrem proximalen Endbereich nachteilig, da hierfür in einem Bereich viel Raum benötigt wird, in dem andernfalls z.B. Antriebskomponenten untergebracht werden könnten. Schliesslich ist bei dieser Vorrichtung nicht sichergestellt, dass die Kolbenstange den Stopfen des Medikamentenreservoirs auch tatsächlich berührt.

### Darstellung der Erfindung

Es ist daher eine Aufgabe der vorliegenden Erfindung, eine Verabreichungsvorrichtung anzugeben, die diese Nachteile zumindest teilweise überwindet.

Diese Aufgaben werden durch eine Vorrichtung mit den Merkmalen des Anspruchs 1 oder des Anspruchs 12 gelöst. Vorteilhafte Weiterbildungen sind in den abhängigen Ansprüchen angegeben.

Gemäss einem ersten Aspekt der vorliegenden Erfindung wird eine Vorrichtung zur Verabreichung eines fluiden Produkts angegeben, die ein Gehäuse und ein Förderelement zum Fördern des Produkts aus einem Reservoir aufweist. Das Förderelement ist relativ zum Gehäuse entlang einer Vorschubachse bewegbar. Anstelle eines Aussengewindes weist das Förderelement ein Innengewinde auf, dessen Gewindeachse entlang der Vorschubachse verläuft. Dadurch wird das zum Antrieb verwendete Gewinde quasi unsichtbar für den Benutzer, und es wird eine weitgehend beliebige, an die Bedürfnisse des Benutzers und andere technische Erfordernisse angepasste Gestaltung der Aussenseite des Förderelements ermöglicht. Zudem wird eine besonders kompakte Anordnung möglich. Die Vorrichtung weist ausserdem eine Antriebseinrichtung sowie ein Gewindeelement mit einem Aussengewinde auf, das mit dem Innengewinde in Eingriff steht und relativ zum Gehäuse entlang der Vorschubachse fixierbar und von der Antriebseinrichtung in eine Drehbewegung um die Vorschubachse versetzbar ist, um das Förderelement entlang der Vorschubachse vorzuschieben.

Das Förderelement ist, anders als im Stand der Technik, jederzeit relativ zum Gehäuse drehfest geführt, vollführt also sowohl bei der Vorschubbewegung zum Ausstossen des Produkts als auch beim Zurückschieben im Rahmen eines Behältniswechsels eine rein lineare Vorschubbewegung.

Das Gewindeelement ist vorzugsweise als Aussengewindestange ausgeführt ist, und das Förderelement weist vorzugsweise nur im Bereich seines proximalen Endes ein im Vergleich zum Aussengewinde des Gewindeelements kurzes Innengewinde auf, um Reibungsverluste zu minimieren.

Das Förderelement kann insbesondere in einer distalen Richtung, d.h. der Richtung, in welcher der Vorschub erfolgt, federbelastet sein. Die dazu dienende Feder ist bevorzugt so schwach, dass sie keinen Ausstoss des Produkts aus dem Reservoir bewirkt. Sie dient vielmehr dazu, bei einem Reservoirwechsel das Förderelement selbsttätig in seine distale Endstellung zu bringen, also vollständig auszufahren, wenn das Reservoir vom Gehäuse entfernt wird. Auf diese Weise wird sichergestellt, dass das Förderelement jederzeit den Reservoirstopfen berührt. Die entsprechende Feder ist vorzugsweise im Inneren des Gewindeelements, das hierzu als Hohlspindel ausgestaltet ist, untergebracht. Es ist vorzugsweise als Schraubenfeder ausgebildet und auf einer Führungsnadel geführt.

In einer bevorzugten Ausgestaltung weist die Vorrichtung ausserdem einen vom Gehäuse lösbaren Behältnishalter zum Halten des Reservoirs mit dem Produkt auf. In einem Zustand, in dem der Behältnishalter vom Gehäuse gelöst ist, ist dann das Gewindeelement vorzugsweise gegenüber dem Gehäuse derart drehbar, dass das Förderelement in diesem Zustand durch eine rein axiale Verschiebung in proximaler Richtung in das Gehäuse einschiebbar ist. Dabei dreht das Gewindeelement aufgrund des Gewindeeingriffs mit dem Förderelement mit.

In einer bevorzugten Ausführungsform ist das Förderelement radial zumindest teilweise von einer Führungshülse mit einem proximalen und einem distalen Ende umgeben. Die Länge dieser Führungshülse entspricht vorzugsweise mindestens dem maximalen Vorschubbereich des Förderelements, d.h. dem Abstand zwischen der distalen und der proximalen Endstellung des Förderelements. Nahe ihrem distalen Ende ist die Führungshülse zumindest während der Verabreichung drehfest mit dem Gehäuse oder einem gehäusefesten Element verbunden. Das Förderelement ist entlang der Vorschubrichtung drehfest in der Führungshülse geführt. Vorzugsweise steht dazu das Förderelement zumindest im Bereich seines proximalen Endes mit dem Inneren der Führungshülse in einem drehfesten Eingriff, der aber eine axiale Verschiebung erlaubt. Dazu kann im Bereich des proximalen Endes eine radial nach aussen aus der äusseren Umfangsfläche des Förderelements vorstehende Eingriffsstruktur, z.B. in Form von mehreren Längsrippen, ausgebildet sein, die mit einer komplementären Struktur, z.B. in der Form von mehreren Längsnuten, an der inneren Umfangsfläche der Führungshülse zusammenwirkt. Die Vorrichtung umfasst dann vorzugsweise ausserdem eine äussere Hülse mit einem proximalen und einem distalen Ende, welche wiederum die Führungshülse zumindest teilweise radial umgibt. Nahe ihrem proximalen Ende ist diese äussere Hülse mit dem drehbaren Element verbunden. Nahe ihrem distalen Ende ist die äussere Hülse drehbar und verschiebegesichert relativ zur Führungshülse geführt.

Die dieser Ausführungsform zu Grunde liegenden Überlegungen lassen sich wie folgt zusammenfassen: Das Förderelement soll relativ zum stationären Gehäuse verdrehgesichert und längsgeführt sein. Dabei soll das Förderelement vorzugsweise auf seiner Aussenseite im Wesentlichen glatt oder auf andere Weise beliebig ausgestaltet sein können. Dies bedingt, dass das Förderelement nur im Bereich seines proximalen Endes in einem zum Gehäuse drehfesten Element längsgeführt ist. Dies wiederum bedingt aber, dass dieses drehfeste Element mindestens so lang ist, dass die Führung auch über den gesamten Bereich des Vorschubs des Förderelements gewährleistet ist. Um dies zu gewährleisten, wird eine hülsenartige Struktur des drehfesten Elements gewählt, das deshalb als Führungshülse bezeichnet wird, mit einer Länge dieser Führungshülse, die mindestens dem Vorschubbereich des Förderelements entspricht. Zum Antrieb des Förderelements muss des weiteren das drehbare Element im Inneren des Förderelements angeordnet sein. Damit ein Gewindeeingriff über den gesamten Vorschubbereich gewährleistet ist, muss sich das drehbare Element zumindest über eine Länge von proximal nach distal in das Förderelement hinein erstrecken, die dem Vorschubbereich entspricht. Der einzige verfügbare Zugang zum drehbaren Element besteht dabei vom proximalen Ende her. Ein Antrieb des drehbaren Elements muss also über dessen proximales Ende erfolgen. Gleichzeitig muss das drehbare Element in irgendeiner Weise relativ zu einem gehäusefesten Element gelagert sein. Diese Lagerung muss in der Lage sein, Axialkräfte aufzunehmen, da Axialkräfte vom Förderelement über den Gewindeeingriff auf das drehbare Element übertragen werden. Zwar könnte eine Lagerung direkt am proximalen Ende des drehbaren Elements vorgesehen werden. Diese würde aber sehr viel Platz in einem Bereich beanspruchen, der nach Möglichkeit für andere Aufgaben zur Verfügung stehen sollte. Es ist also wünschenswert, den Ort der Lagerung an eine andere Stelle, möglichst nahe einem der Enden des Gehäuses, zu verlegen. Dies geschieht hier mit Hilfe der äusseren Hülse, die fest mit dem drehbaren Element verbunden ist und dieses quasi entlang der Aussenseite der Führungshülse zur distalen Richtung hin fortsetzt. Die Lagerung erfolgt dann zwischen der äusseren Hülse und der Führungshülse, und zwar im Bereich des distalen Endes der äusseren Hülse. Dadurch wird eine sehr kompakte Einheit geschaffen, deren radial relativ viel Platz beanspruchende Lagerung in einem proximalen Bereich zu liegen kommt, wo sie nur wenig stört, und die es ermöglicht, im radialen Zwischenraum zwischen der äusseren Hülse und dem Gehäuse weitere Elemente des Injektionsgeräts unterzubringen. Des weiteren ermöglicht die Tatsache, dass die äussere Hülse (und damit letztlich das drehbare Element) an der Führungshülse und nicht etwa am Gehäuse selbst gelagert ist, dass die gesamte Einheit aus Führungshülse, äusserer Hülse, Lagerung, drehbarem Element und Förderelement gegenüber dem Gehäuse gezielt bewegbar ausgestaltet werden kann, z.B. im Rahmen eines Behältniswechsels.

Gemäss einem zweiten Aspekt der vorliegenden Erfindung umfasst eine Vorrichtung zur Verabreichung eines fluiden Produkts ein Gehäuse und ein Förderelement zum Fördern des Produkts aus einem Reservoir. Das Förderelement ist relativ zum Gehäuse entlang einer Vorschubachse zwischen einer proximalen Ausgangsstellung und einer distalen Endstellung bewegbar und bevorzugt drehfest geführt. Es ragt dabei durch eine Durchtrittsöffnung in einer Begrenzungswand des Gehäuses hindurch. Des weiteren ist eine Antriebseinrichtung zum Erzeugen einer Drehbewegung eines drehbaren Elements relativ zum Gehäuse vorhanden, wobei das drehbare Element so mit dem Förderelement gekoppelt ist, dass die Drehbewegung eine Vorschubbewegung des Förderelements entlang der Vorschubachse bewirkt. Das Förderelement weist einen im Wesentlichen glatten Aussenwandbereich auf, dessen Länge mindestens dem Abstand zwischen der distalen Endstellung und der proximalen Ausgangsstellung entspricht. Auf diese Weise wird es möglich, das Förderelement so anzuordnen, dass während des Vorschubs nur der glatte Aussenwandbereich durch die Öffnung in der Gehäusewand hindurch vorgeschoben wird. So kann vermieden werden, dass der Benutzer ein Aussengewinde zu sehen bekommt, das eine abschreckende Wirkung auf ihn haben könnte oder das schwer zu reinigen sein könnte.

Unter einem "im Wesentlichen glatten" Aussenwandbereich ist insbesondere ein Bereich der äusseren Umfangsfläche des Förderelements zu verstehen, der keine technisch bedingten Strukturen aufweist, die zu einer ungenügenden Akzeptanz durch den Benutzer führen könnten oder die Reinigung erschweren könnten. Andererseits kann der Aussenwandbereich aber durchaus feine Strukturen aufweisen, z.B. eine Mikro- oder Nanostrukturierung. Distal vom glatten Aussenwandbereich kann sich z.B. eine Struktur anschliessen, die dazu ausgebildet ist, einen Stopfen im Produktbehälter vorzuschieben, z.B. einen radial über den Aussenwandbereich vorstehenden Vorschubflansch. Der proximal vom Aussenwandbereich angeordnete Bereich befindet sich dagegen normalerweise permanent innerhalb des Gehäuses und ist damit für den Benutzer unsichtbar. Dieser proximale Bereich kann daher im Rahmen der technischen Anforderungen beliebig ausgestaltet sein.

Falls der Vorschub des Förderelements über ein Aussengewinde erfolgen soll, auf dem in herkömmlicher Art eine Antriebsmutter läuft, so wird sich dieses Aussengewinde typischerweise proximal an den glatten Aussenwandbereich anschliessen.

In einer vorteilhaften Ausführungsform ist das Förderelement gegenüber dem Gehäuse oder einem gehäusefesten Element flüssigkeitsdicht, mindestens spritzwassergeschützt, abgedichtet. Dadurch wird ein Eindringen von Flüssigkeiten wirksam verhindert.

Eine entsprechende Dichtung kann unmittelbar zwischen dem Förderelement und dem Gehäuse ausgebildet sein. Es ist aber auch denkbar, dass eine erste Dichtung zwischen dem Förderelement und einem Element, das relativ zum Gehäuse beweglich ist, ausgebildet ist, und eine zweite Dichtung zwischen dem beweglichen Element und dem Gehäuse ausgebildet ist. Das bewegliche Element kann z.B. so angeordnet sein, dass es nur bei einem Wechsel des Reservoirs bewegbar ist und während der eigentlichen Verabreichung zum Gehäuse unbeweglich ist. Es kann insbesondere hülsenförmig sein.

Bevorzugt ist ein im Wesentlichen glatter, zylindrischer, bevorzugt kreiszylindrischer Aussenwandbereich von wenigstens einem ringförmigen Dichtelement umgeben. Auf diese Weise kann eine einfache und effiziente Abdichtung erreicht werden, wie sie bei den Kolbenstangen mit Aussengewinde des Standes der Technik nicht möglich ist. Die Länge des im Wesentlichen glatten Aussenwandbereichs entspricht dabei mindestens dem Abstand zwischen der distalen Endstellung und der proximalen Ausgangsstellung, zwischen denen das Förderelement im Verlaufe der Verabreichung bewegbar ist, um eine Abdichtung über diesen gesamten Bereich sicherzustellen. Der Aussenwandbereich kann feine Strukturen, insbesondere im Bereich unterhalb von 100 Mikrometern, bevorzugt unterhalb von 10 Mikrometern aufweisen, die geeignet sind, die Dichtwirkung zumindest nicht zu beeinträchtigen oder gar zu verbessern, z.B. eine Mikro- oder Nanostrukturierung. Diese Strukturen können eine Vorzugsrichtung aufweisen, um ein Fliessen von Flüssigkeiten zu hemmen.

Das wenigstens eine ringförmige Dichtelement umfasst bevorzugt wenigstens eine umlaufende, biegsame Dichtlippe, die auf dem zylindrischen Aussenwandbereich aufliegt und nach Art eines Scheibenwischers als Abstreifer wirkt. Der Kontaktwinkel zwischen der Dichtlippe und dem Aussenwandbereich beträgt dabei bevorzugt weniger als 90 Grad. Es kann aber z.B. auch als Ring mit rundem oder vieleckigen Querschnitt ausgebildet sein.

Bevorzugt ist das Innere des Gehäuses vollständig gegenüber dem Äusseren des Gehäuses flüssigkeitsdicht abgedichtet, also nicht nur im Bereich, in dem das Förderelement nach aussen geführt ist, sondern auch in anderen Bereichen, z.B. im Bereich von beweglichen Dosier- oder Betätigungselementen.

Das Förderelement oder ein mit diesem dichtend zusammenwirkendes Element kann insbesondere aus einem hydrophoben Material bestehen oder hydrophob beschichtet sein, um eine Dichtwirkung zu erreichen oder zu verbessern, indem ein Kriechen durch Kapillarwirkung vermieden wird.

Das Reservoir wird bevorzugt in einem Behältnishalter gehalten, der lösbar am Gehäuse befestigbar ist. Beim Befestigen ist dieser vorzugsweise derart gegenüber dem Gehäuse geführt, dass er relativ zum Gehäuse eine kombinierte Drehbewegung um eine Drehachse und Translationsbewegung entlang der Drehachse ausführt. Die Vorrichtung umfasst dann bevorzugt des weiteren ein Federelement und ein Rastelement. Diese Elemente sind derart angeordnet, dass das Federelement eine im Wesentlichen entlang der Drehachse wirkende Federkraft auf das Rastelement erzeugt. Das Rastelement bewirkt dadurch in einer vorbestimmten Haltestellung des Behältnishalters eine lösbare Rastverbindung, durch die der Behältnishalter relativ zum Gehäuse fixiert wird. Dazu kann beispielsweise in Vorsprung des Rastelements in eine Vertiefung des Behältnishalters oder eines dazu drehfesten Elements hineinragen. Auch eine umgekehrte Anordnung ist denkbar. Die Rastverbindung ist bevorzugt so ausgelegt, dass sie durch eine Bewegung des Behältnishalters relativ zum Gehäuse, die der Bewegung beim Befestigen entgegengesetzt ist, wieder lösbar ist, d.h., die Rastverbindung braucht nicht manuell entriegelt zu werden.

Es ist also vorzugsweise eine Verbindung nach Art einer Schraub- oder bevorzugt Bajonettverbindung zwischen dem Behältnishalter und dem Gehäuse bzw. zwischen mit diesen verbundenen Elementen vorgesehen. Bei üblichen Bajonettverbindungen zwischen zwei Elementen wird in der Regel ein Element mit einem Bajonettzapfen in einem geeignet geformten Schlitz oder in einer entsprechenden Nut nach Art eine Kulissenführung des anderen Elements geführt, bis der Bajonettzapfen eine Endstellung (entsprechend der Haltestellung) erreicht. Bei solchen Verbindungen besteht das Problem, ein unbeabsichtigtes Zurückgleiten des Zapfens aus der Endstellung heraus zu vermeiden. Ein ähnliches Problem stellt sich bei Schraubverbindungen, die durch eine Klemmkraft fixiert werden und sich ebenfalls unbeabsichtigt lösen können. Die vorliegende Ausgestaltung löst dieses Problem, indem sie ein axial federbelastetes (und axial bewegbares) Rastelement vorsieht. Ein solches Rastelement gewährleistet eine definierte Fixierung des Behältnishalters in der Haltestellung, ohne dass die Gefahr eines unbeabsichtigten Lösens besteht. Die Verbindung ist nur durch Überwindung einer genügend grossen Kraft wieder lösbar. Dabei wird ein Verschleiss, der zu einem Ausleiern der Verbindung führen könnte, vermieden.

Indem das Rastelement in die axiale Richtung federbelastet ist, wird eine einfache und platzsparende Ausgestaltung des Rastelements und des Federelements ermöglicht.

In der Regel wird es sich bei dem Behältnis um einen Behälter mit einem zylindrischen Wandbereich und einem darin verschiebbaren Stopfen, eine sogenannte Karpule, handeln, der eine Längsrichtung definiert und in einem Behältnishalter mit entsprechend langgestreckter Form untergebracht ist. Beim Befestigen am Gehäuse wird der Behältnishalter vorzugsweise um diese Längsrichtung gedreht, d.h., die Drehachse fällt mit der Längsachse zusammen. Zur Verabreichung des Produkts wird der Stopfen durch das Förderelement entlang der Längsrichtung vorgeschoben. Allgemeiner ausgedrückt, entspricht somit vorzugsweise die Vorschubrichtung des Förderelements einer Richtung entlang der Drehachse, um die der Behältnishalter bei seiner Befestigung gedreht wird.

Für die Anordnung des Feder- und Rastelements bestehen verschiedene Möglichkeiten. Gemäss einer bevorzugten Ausgestaltung ist das Rastelement relativ zum Gehäuse drehfest und in der Raststellung mit einem Element lösbar verrastet, das relativ zum Behältnishalter drehfest ist. In anderen Worten folgt das Rastelement nicht der Drehung des Behältnishalters, sondern bleibt bei der Befestigung des Behältnishalters drehfest zum Gehäuse. Jedoch ist auch die umgekehrte Anordnung denkbar, bei der das Rastelement relativ zum Behältnishalter drehfest ist.

In einer bevorzugten Ausführungsform umfasst die Vorrichtung ein Mitnahmeelement, das relativ zum Gehäuse drehbar angeordnet ist. Das Mitnahmeelement ist derart gegenüber dem Gehäuse geführt, dass es beim Befestigen des Behältnishalters am Gehäuse sowie beim Lösen des Behältnishalters vom Gehäuse durch den Behältnishalter mitgenommen und in eine Bewegung versetzt wird, die eine Drehbewegung um die Drehachse umfasst. Das Rastelement bewirkt dann in der Haltestellung des Behältnishalters eine lösbare Rastverbindung, durch die das Mitnahmeelement relativ zum Gehäuse fixiert wird. Dieses wiederum hält dann den Behältnishalter. Der Behältnishalter wird also indirekt, über das Mitnahmeelement, relativ zum Gehäuse fixiert. Dadurch wird eine grössere Freiheit bei der Gestaltung des Behältnishalters und des Rastelements und Federelements ermöglicht.

In einer vorteilhaften Ausgestaltung umfasst die Vorrichtung ein relativ zum Gehäuse drehfest angeordnetes Führungselement, das insbesondere als Führungshülse ausgebildet sein kann. Dieses Führungselement kann starr mit dem Gehäuse verbunden sein, insbesondere einstückig mit diesem gefertigt sein, oder es kann axial gegenüber dem Gehäuse verschiebbar sein. Das Mitnahmeelement, das ebenfalls als Hülse ausgestaltet sein kann und dann als Bajonetthülse bezeichnet werden kann, ist mit dem Führungselement mindestens drehbar verbunden. Das Federelement und das Rastelement sind vorzugsweise relativ zum Führungselement drehfest angeordnet, und das Rastelement ist in der Haltestellung des Behältnishalters mit dem Mitnahmeelement lösbar verrastet. Insbesondere kann das Führungselement gegenüber dem Gehäuse axial verschiebbar und das Mitnahmeelement zwar drehbar, aber axial verschiebefest mit dem Führungselement verbunden sein. Durch diese Gestaltung wird ermöglicht, dass weitere im Gehäuse angeordnete Teile der Vorrichtung, die mit dem Führungselement verbunden sind, axial verschoben werden, wenn der Behältnishalter am Gehäuse angebracht wird. Dadurch kann die Verabreichungsvorrichtung automatisch beim Lösen des Behältnishalters z.B. zurückgesetzt und beim Befestigen des Behältnishalters wieder in einen betriebsbereiten Zustand gebracht werden. Insbesondere ist es auf diese Weise möglich, beim Lösen des Behältnishalters das Innere der Vorrichtung (insbesondere die Antriebseinrichtung und gegebenenfalls eine damit verbundene Auslöseeinrichtung) so in eine distale Richtung zu verschieben, dass die Auslöseeinrichtung, die als Druckknopf ausgestaltet sein kann, in das Gehäuse eingezogen wird und so anzeigt, dass die Vorrichtung nicht betriebsbereit ist.

Bevorzugt ist dabei das Mitnahmeelement derart gegenüber dem Gehäuse geführt, dass es beim Befestigen des Behältnishalters am Gehäuse vom Behältnishalter mitgenommen und relativ zum Gehäuse in eine kombinierte Drehbewegung um die Drehachse und Translationsbewegung entlang der Drehachse in einer proximalen Richtung versetzt wird.

Vorzugsweise ist das Mitnahmeelement zwischen zwei definierten Endstellungen bewegbar, und das Rastelement bewirkt in *beiden* Stellungen eine lösbare Rastverbindung, durch die das Mitnahmeelement relativ zum Gehäuse fixiert wird. Dabei nimmt das Mitnahmeelement seine erste Endstellung ein, wenn der Behältnishalter seine Haltestellung einnimmt, und es nimmt seine zweite Endstellung ein, wenn der Behältnishalter vom Gehäuse abgenommen ist. Bevorzugt ist das Rastelement sowohl in der ersten Endstellung als auch in der zweiten Endstellung unmittelbar mit dem Mitnahmeelement lösbar verrastet.

Das Mitnahmeelement ist vorzugsweise in mindestens einem Führungsschlitz relativ zum Gehäuse, d.h. am eigentlichen Gehäuse selbst oder an einem gehäusefesten Element, geführt, insbesondere durch ein oder mehrere entsprechende Zapfen. Ebenso ist der Behältnishalter beim Befestigen am Gehäuse in mindestens einem Führungsschlitz, nach Art einer Bajonettverbindung, relativ zum Gehäuse geführt.

Das Rastelement ist vorzugsweise in Form eines Rings ausgebildet, der sich um die Drehachse und insbesondere um das Vorschubelement herum erstreckt. Auf dem Ring können geeignete Rastnasen ausgebildet sein. Dieses Rastelement kann durch ein getrenntes Federelement axial federbelastet sein. Dabei kann es sich z.B. um eine auf Druck belastete Schraubenfeder oder eine andere Art von elastischem Element handeln. Bevorzugt weist das Federelement jedoch die Form eines Rings auf, welcher sich um die Drehachse herum erstreckt und um eine Achse senkrecht zur Drehachse gekrümmt ist, so dass durch ein Zusammenpressen des Federelements entlang der Drehachse die Federkraft erzeugt wird.

Bevorzugt ist das Rastelement einstückig mit dem Federelement ausgebildet.

Dies führt zu einer besonders einfachen Ausgestaltung. Insbesondere kann das Rastelement als ein in Richtung der Drehachse vorstehender Vorsprung auf dem Federelement ausgebildet sein. Dies ist insbesondere dann von Vorteil, wenn das Federelement, wie oben beschrieben, die Form eines gekrümmten Rings aufweist.

Die Vorrichtung kann des weiteren mindestens ein Kugellager aufweisen, welches Kräfte aufnimmt, die zwischen dem Förderelement und dem Gehäuse übertragen werden. Indem ein Kugellager vorgesehen wird, werden Reibungsverluste, die während der Verabreichung aufgrund der Übertragung von Kräften zwischen dem Förderelement und dem Gehäuse verursacht werden, weitgehend minimiert.

Ein Kugellager ist insbesondere dann vorteilhaft, wenn die Vorrichtung mindestens ein mit dem Förderelement zusammenwirkendes drehbares Element und eine Antriebseinrichtung zur Erzeugung einer Drehbewegung des drehbaren Elements relativ zum Gehäuse umfasst, wobei die Drehbewegung des drehbaren Elements eine (vorzugsweise lineare) Vorschubbewegung des Förderelements entlang einer Vorschubachse in einer distalen Richtung bewirkt, vorzugsweise durch einen Gewindeeingriff, der bevorzugt nicht blockierend ist. In diesem Fall ist das Kugellager vorteilhaft derart angeordnet, dass es entlang der Vorschubachse wirkende (Axial-) Kräfte aufnimmt, welche vom (vorzugsweise relativ zum Gehäuse drehfesten) Förderelement über das drehbare Element auf das Gehäuse übertragen werden, also zwischen dem drehbaren Element und dem Gehäuse wirken. Allgemein ausgedrückt werden also axiale, insbesondere in proximaler Richtung wirkende Kräfte zwischen dem Förderelement und dem Gehäuse über eine drehbare und in axialer Richtung verschiebefeste Verbindung hinweg übertragen. Im Stand der Technik wird hierzu in der Regel eine gleitende Verbindung zwischen dem drehbaren Element und dem Gehäuse bzw. einem gehäusefesten Element vorgesehen. Bei der vorliegenden Ausgestaltung ist dagegen mindestens ein Kugellager vorgesehen, das bevorzugt zwischen dem drehbaren Element oder einem damit verbundenen Element einerseits und dem Gehäuse oder einem zumindest während der Verabreichung gehäusefesten Element andererseits angeordnet ist. Dadurch können Reibungsverluste bei der Drehung des drehbaren Elements vermieden werden.

Bevorzugt sind zwei Kugellager vorhanden, die vorzugsweise so angeordnet sind, dass sie Kräfte entlang der Vorschubachse sowohl in einer proximalen Richtung als auch in der dazu entgegengesetzten distalen Richtung, d.h. der Richtung, in welcher der Vorschub erfolgt, aufnehmen können. Axialkräfte in der proximalen Richtung entstehen in der Regel während der Verabreichung, während Axialkräfte in der distalen Richtung insbesondere dann entstehen können, wenn das Förderelement in der distalen Richtung federbelastet ist, wie dies in einer bevorzugten Ausführungsform der Fall ist. Die dazu dienende Feder ist bevorzugt so schwach, dass sie keinen Ausstoss des Produkts aus dem Reservoir bewirkt. Sie dient vielmehr dazu, bei einem Reservoirwechsel das Förderelement selbsttätig in seine distale Endstellung zu bringen, also vollständig auszufahren, wenn das Reservoir vom Gehäuse entfernt wird. Wenn das Förderelement mit dem drehbaren Element geeigneter Verbindung steht, führt dies dazu, dass das drehbare Element in eine Drehung versetzt wird, wenn das Förderelement aufgrund der Federkraft ausfährt. Um die dazu nötige Federkraft klein halten zu können, ist ein Kugellager von besonderem Vorteil, da es beim Ausfahren des Förderelements die dabei wirkenden Reibungskräfte minimiert.

### Kurze Beschreibung der Zeichnungen

Bevorzugte Ausführungsformen der Erfindung werden im Folgenden anhand der Zeichnungen beschrieben, in denen zeigen:
- Fig. 1: eine perspektivische Explosionsansicht eines Injektionsgeräts gemäss einer ersten Ausführungsform;
- Fig. 2: einen Längsschnitt durch das Injektionsgerät der Fig. 1;
- Fig. 3: einen vergrösserten Ausschnitt aus der Fig. 2;
- Fig. 4: einen Längsschnitt durch eine Anordnung einer Führungshülse und einer Kupplungshülse im Injektionsgerät der Fig. 1;
- Fig. 5A: eine Draufsicht auf einen Kugellagerring;
- Fig. 5B: eine Schnittansicht des Kugellagerrings in der Ebene A-A;
- Fig. 6: eine perspektivische Ansicht einer Kupplungshülse;
- Fig. 7: einen Längsschnitt durch ausgewählte Teile des Injektionsgeräts der Fig.1;
- Fig. 8: eine perspektivische Darstellung einer Bajonetthülse;
- Fig. 9A: eine Draufsicht auf eine Bajonettfeder;
- Fig. 9B: eine Seitenansicht der Bajonettfeder der Fig. 8A;
- Fig. 9C: eine perspektivische Ansicht der Bajonettfeder der Fig. 8A;
- Fig. 10: einen Längsschnitt durch ausgewählte Teile des Injektionsgeräts der Fig. 1;
- Fig. 11A: einen Längsschnitt durch ausgewählte Teile des Injektionsgeräts der Fig. 1 mit einem Dosisbegrenzungsring in seiner Endstellung;
- Fig. 11B: einen Längsschnitt durch ausgewählte Teile des Injektionsgeräts der Fig. 1 mit dem Dosisbegrenzungsring in seiner Ausgangsstellung;
- Fig. 12A: eine perspektivische Ansicht einer Kupplungswelle mit einem Dosisbegrenzungsring des Injektionsgeräts der Fig. 1;
- Fig. 12B: die Teile der Fig. 13A in einer Explosionsansicht;
- Fig. 12C: die Kupplungswelle der Fig. 13A in einer perspektivischen Ansicht aus einer anderen Blickrichtung;
- Fig. 13: eine Explosionsansicht einer Arretierhülse, einer Kupplungsfeder und eines Stützrings;
- Fig. 14: eine perspektivische Ansicht ausgewählter Teile des Injektionsgeräts der Fig. 1;
- Fig. 15: eine perspektivische Explosionsansicht des proximalen Endes des Injektionsgeräts der Fig. 1;
- Fig. 16: einen Längsschnitt durch das Injektionsgerät der Fig. 1 in der Ausgangsstellung;
- Fig. 17: den Längsschnitt der Fig. 16 bei eingedrücktem Druckknopf;
- Fig. 18: den Längsschnitt der Fig. 16 nach einem ersten Aufdosieren bis zur halben Maximaldosis;
- Fig. 19: den Längsschnitt der Fig. 16 nach einem ersten Aufdosieren bis zur vollen Maximaldosis;
- Fig. 20: den Längsschnitt der Fig. 16 nach einem ersten Auslösen und einem zweiten Aufdosieren;
- Fig. 21: einen Längsschnitt durch das Injektionsgerät der Fig. 1 nach vollständiger Entleerung der Karpule;
- Fig. 22: eine perspektivische Ansicht des Injektionsgeräts der Fig. 1 nach Entfernung der Karpulenhülse; sowie
- Fig. 23: einen Längsschnitt durch ein Injektionsgerät gemäss einer zweiten Ausführungsform.

### Ausführliche Beschreibung bevorzugter Ausführungsformen

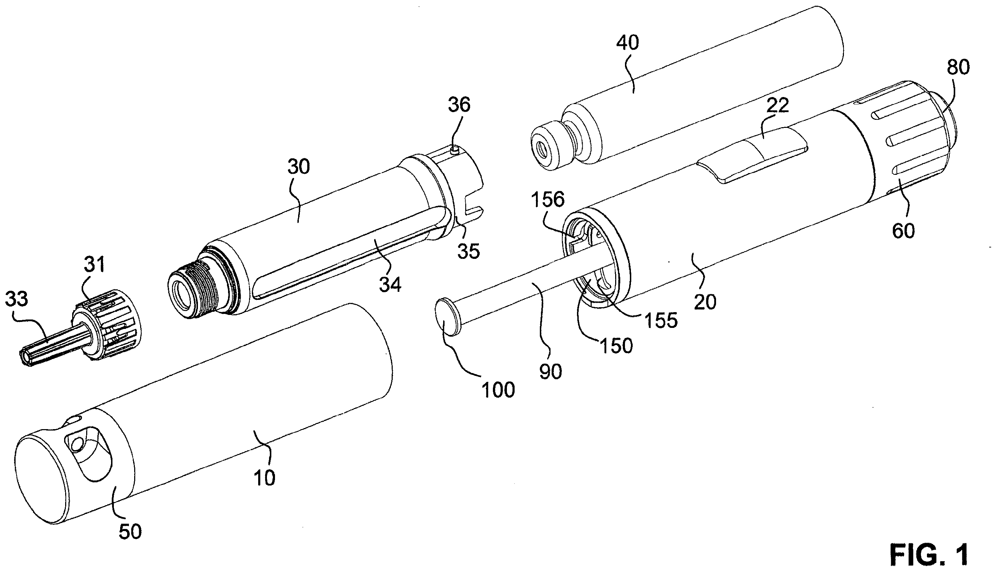

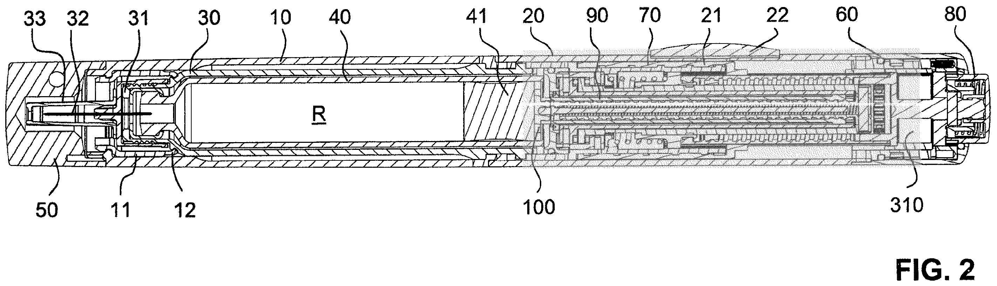

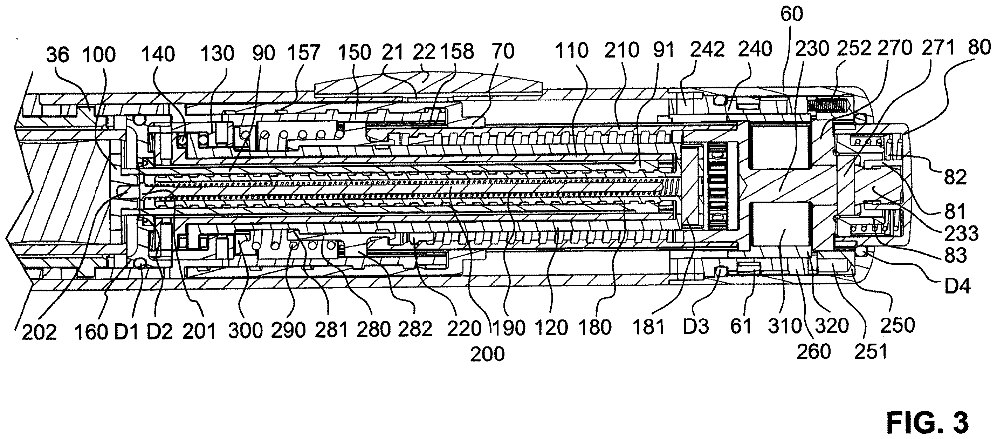

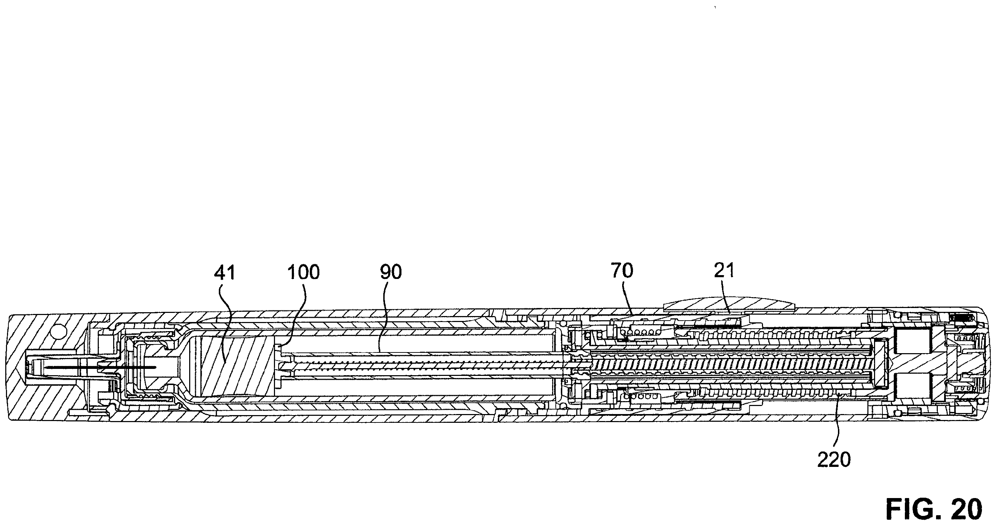

Anhand der Figuren 1 und 2 werden im Folgenden zunächst der grundsätzliche Aufbau und die grundsätzliche Bedienung eines erfindungsgemässen Injektionsgeräts erläutert, bevor für eine erste Ausführungsform der Aufbau des Geräts und die Funktionsweise der Dosiermechanik im Einzelnen anhand der Figuren 3 bis 19 erläutert wird. Eine zweite Ausführungsform wird dann anhand der Fig. 20 diskutiert.

### Grundsätzlicher Aufbau und Bedienung

Die Fig. 1 zeigt ein Injektionsgerät in Form eines Injektionspens in einer perspektivischen Explosionsansicht. Die Fig. 2 zeigt das Gerät im Längsschnitt. Die folgende Beschreibung bezieht sich auf das Gerät im zusammengesetzten Zustand, wie es in der Fig. 2 dargestellt ist.

Das Injektionsgerät weist eine Gehäusehülse 20 auf, in der eine Mechanik zum Einstellen und Ausschütten einer Dosis untergebracht ist. Die Gehäusehülse 20 ist im Wesentlichen kreiszylinderförmig und definiert hierdurch eine Längsachse. Über eine unten näher beschriebene Bajonettverbindung ist ein Behältnishalter in Form einer Karpulenhülse 30 an einem distalen Ende der Gehäusehülse 20 lösbar befestigt. Diese nimmt ein Behältnis in Form einer Karpule 40 mit einem flüssigen Medikament auf, in der ein Stopfen 41 verschiebbar geführt ist. Dadurch wird ein Medikamentenreservoir R veränderlichen Volumens innerhalb der Karpule begrenzt. Statt einer Karpule kann auch ein anderes Behältnis vorhanden sein, dessen Volumen veränderlich ist, z.B. ein Behältnis mit ziehharmonikaartig gefalteten Wänden nach Art eines Faltenbalgs. Durch ein langgestrecktes Sichtfenster 34 in der Karpulenhülse 30 kann der Inhalt der Karpule 40 kontrolliert werden. Am distalen Ende der Karpulenhülse 30 ist ein Nadelhalter 31 aufgeschraubt, der eine als Injektionsnadel dienende Hohlnadel (Kanüle) 32 trägt, deren proximales Ende durch ein dichtendes Septum hindurch in das Medikamentenreservoir R ragt. Eine abziehbare Nadelschutzhülle 33 umgibt den nach vorne ragenden Bereich der Nadel 32 und schützt einen Benutzer vor einem versehentlichen Einstechen. Eine Schutzhülse 10, deren distales Ende permanent mit einer Schutzkappe 50 verschlossen ist, ist über die Karpulenhülse 30 geschoben. Im Inneren der Schutzhülse 10 ist ein Haltering 11 mit sich in proximaler Richtung erstreckenden Rastarmen 12 montiert. Die Enden der Rastarme 12 sind lösbar mit der Karpulenhülse 30 verrastet. Während die Erfindung hier anhand eines Injektionsgeräts erläutert wird, das eine Nadel 32 aufweist, ist es auch denkbar, dass das Injektionsgerät mehrere Nadeln oder keine Nadel wie bei einem Jet-Injektor aufweist.

Am proximalen Ende der Gehäusehülse 20 ist eine Dosierhülse 60 mit darin gehaltenem Druckknopf 80 verdrehbar angeordnet. Die Dosierhülse dient zur Einstellung einer aus dem Medikamentenreservoir R auszuschüttenden Dosis und zum Spannen einer Antriebseinrichtung mit einem Antriebselement in Form einer zeichnerisch nur angedeuteten, als Drehfeder wirkenden Spiralfeder 310. Die eingestellte Dosis wird auf einer Anzeigetrommel 70 angezeigt und kann durch ein Fenster 21 in der Gehäusehülse 20 abgelesen werden, welches durch eine transparente Abdeckung 22 abgedeckt ist. Ein Korrigieren (Verringern) der eingestellten Dosis ist auf einfache Weise durch ein Zurückdrehen der Dosierhülse 60 möglich. Unten wird unter Bezugnahme auf die weiteren Figuren noch im Einzelnen erläutert, wie diese Einstellung ermöglicht wird.

Unter Bezugnahme auf diese Teile lassen sich die folgenden Richtungen definieren, auf die im Folgenden immer wieder Bezug genommen wird: Die distale Richtung ist diejenige Richtung, in der die Verabreichung erfolgt, d.h. sie weist entlang der Längsachse vom Druckknopf 80 in Richtung der Hohlnadel 32. Die proximale Richtung ist entsprechend als die entgegengesetzte Richtung definiert. Falls auf einen Drehsinn (Uhrzeigersinn, Gegenuhrzeigersinn) Bezug genommen wird, so ist damit der Drehsinn gemeint, den man wahrnimmt, wenn man entlang der Längsachse in distaler Richtung blickt.

Nach dem Einstellen der Dosis wird die Hohlnadel 32 durch die Haut des Patienten eingestochen, und es wird eine Ausschüttung der Dosis ausgelöst, indem der Benutzer den Druckknopf 80 in die Dosierhülse 60 eindrückt. Über einen unten im Einzelnen beschriebenen Mechanismus wird durch die Antriebseinrichtung eine Drehbewegung erzeugt, die in einen Vortrieb eines Förderelements in Form einer Vorschubhülse 90 in distaler Richtung umgewandelt wird. Die Vorschubhülse 90 schiebt über einen an ihrem distalen Ende angeordneten Vorschubflansch 100 den Stopfen 41 der Medikamentenkarpule 40 um den eingestellten Betrag in distaler Richtung, wodurch die Ausschüttung des Medikaments aus dem Reservoir R bewirkt wird. Die Vorschubhülse 90 wirkt also als Kolbenstange für den durch den Vorschubflansch 100 und den Stopfen 41 gebildeten Kolben. Nach dem Ende der Verabreichung lässt der Benutzer den Druckknopf 80 wieder los. Die Anzeigetrommel wird während des Vortriebs der Vorschubhülse 90 von der Antriebseinrichtung derart mitgenommen, dass sie im Verlauf der Ausschüttung in ihre Nullstellung zurückkehrt. Damit ist der Injektionspen sofort für die nächste Dosiseinstellung bereit.

Wenn das Medikament im Medikamentenreservoir R zur Neige geht, also die Vorschubhülse 90 beinahe vollständig ausgefahren ist, wird dies durch eine unten näher beschriebene Dosisbegrenzungseinrichtung im Injektionspen erkannt. Die Dosisbegrenzungseinrichtung erlaubt es dem Benutzer dann lediglich noch, maximal die verbleibende verfügbare Restdosis einzustellen. Bei einem anschliessenden Karpulenwechsel stellen sich die Dosisbegrenzungseinrichtung wie auch die Anzeigetrommel 70 automatisch in den Ausgangszustand zurück. Es ist also keine manuelle Rückstellung erforderlich.

Im Folgenden werden Aufbau und Funktionsweise der Mechanik im Einzelnen beschrieben.

### Aufbau der Mechanik

Die Fig. 3 zeigt eine vergrösserte Darstellung des hinteren (proximalen) Bereichs des Injektionsgeräts der Fig. 1. Der Aufbau dieses Bereichs wird nun im Wesentlichen von innen nach aussen im Detail beschrieben.

Die Vorschubhülse 90 ist in einer zum Gehäuse drehfest und verschiebbar angeordneten Führungshülse 110 wiederum drehfest und in Längsrichtung verschiebbar gelagert. Hierzu weist die Vorschubhülse 90 an ihrem proximalen Ende mehrere radial nach aussen ragende Führungsnocken 91 auf, die in dazu komplementären Längsnuten auf der Innenseite der Führungshülse 110 geführt sind.

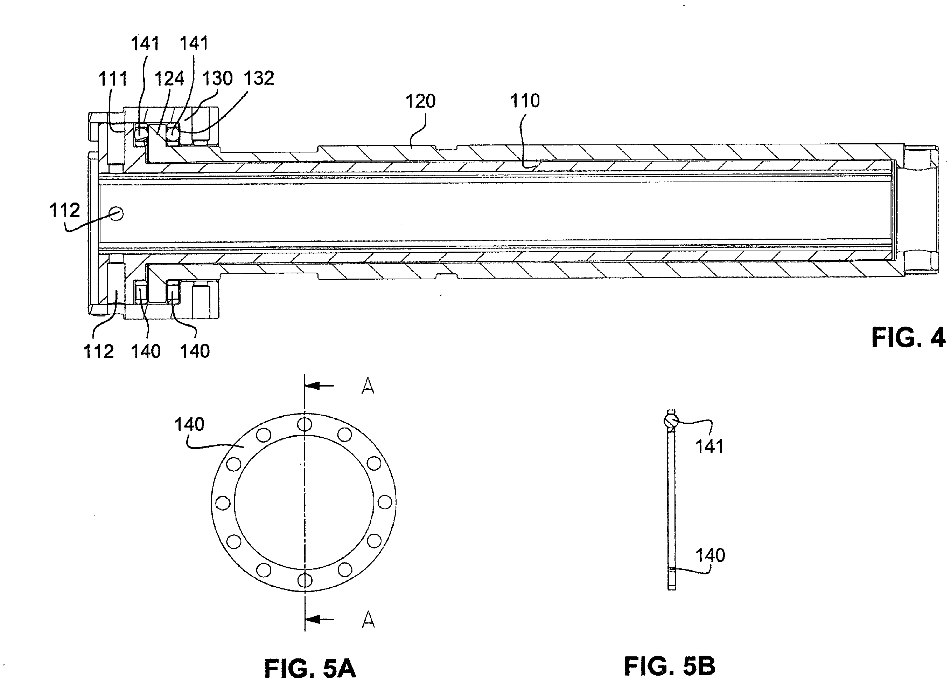

Die Führungshülse 110 ist besonders gut in der Fig. 4 erkennbar, die das Zusammenwirken der Führungshülse mit weiteren Teilen illustriert. Die Führungshülse 110 weist an ihrem distalen Ende einen radial nach aussen ragenden, umlaufenden Ringflansch 111 mit radialen Bohrungen 112 auf. Ein ringförmiger Lagerhalter 130 ist von der proximalen Seite her über die Führungshülse 110 geschoben, umgibt den Ringflansch 111 radial und ist über zeichnerisch nicht dargestellte radiale Zylinderstifte starr mit diesem verbunden.

Eine Kupplungshülse 120 ist zwischen dem Ringflansch 111 der Führungshülse 110 und einer nach innen ragenden Schulter 132 des Lagerhalters 130 drehbar gelagert. Wie unten noch näher beschrieben wird, ist die Kupplungshülse 120 über eine Gewindestange 180 mit der Vorschubhülse 90 verbunden und bildet daher einen Teil einer Fördereinrichtung, die durch eine Drehbewegung angetrieben wird und einen Vorschub des Förderelements in Form der Vorschubhülse bewirkt. Die Kupplungshülse 120 nimmt daher im Betrieb erhebliche Axialkräfte auf, die über ihre Lagerung auf die Führungshülse 110, den Lagerhalter 130, den Mechanikhalter 150 und damit letztlich auf das Gehäuse übertragen werden.

Um die Lagerung möglichst verlustarm zu gestalten, erfolgt die Lagerung über Kugellager. Hierzu ist zwischen dem Flansch 111 der Führungshülse und einem radial umlaufenden Flansch 124 der Kupplungshülse 120 ein erster Kugellagerring 140 vorgesehen. Ein weiterer derartiger Kugellagerring 140 ist zwischen dem Flansch 124 und einer Stirnfläche des Lagerhalters 130 angeordnet.

Der Kugellagerring 140 ist in den Figuren 5A und 5B im Detail dargestellt. Er trägt eine Mehrzahl von Lagerkugeln 141 (hier zwölf Stück). Diese laufen, wie in der Fig. 3 erkennbar ist, in flachen, kreisförmigen Rillen, die in beiden Stirnseiten des radialen Flansches 124 der Kupplungshülse 120, in der entsprechenden Stirnseite des Flansches 111 der Führungshülse 110 sowie in der Stirnseite des Lagerhalters 130 ausgebildet sind.

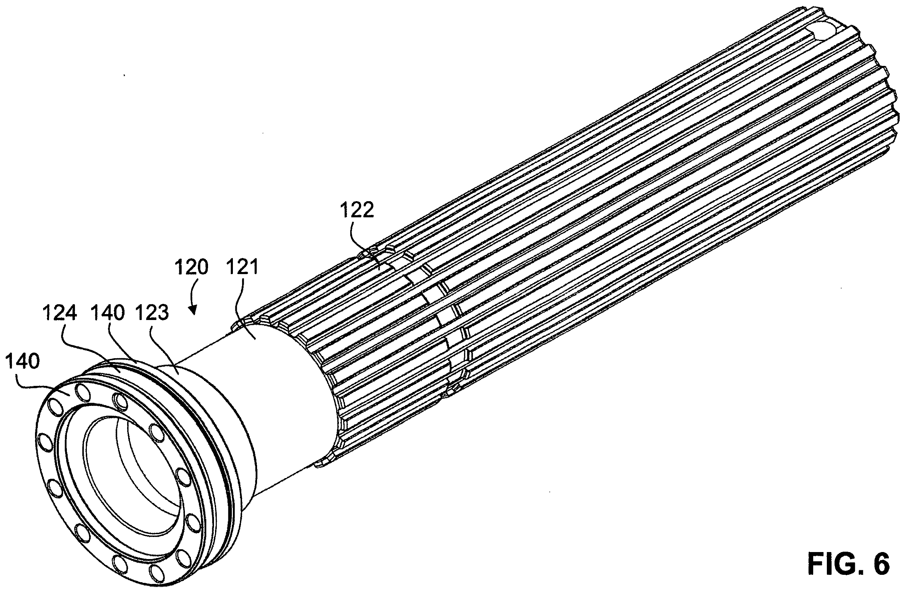

Die Kupplungshülse 120 ist zusammen mit den Kugellagerringen 140 (aber ohne Kugeln 141) in der Fig. 6 dargestellt. Wie hier besonders gut erkennbar ist, sind auf dem zylindrischen Hülsenkörper 121 eine Mehrzahl von Längsrippen 122 ausgebildet, die sich über einen erheblichen Teil der Länge des Hülsenkörpers in Längsrichtung bis zu dessen proximalem Ende erstrecken. Dazwischen sind entsprechende Nuten vorhanden. Auf eine Verdickung 123 folgt nach vorne hin der Flansch 124, der beidseitig an die Kugellagerringe 140 angrenzt.

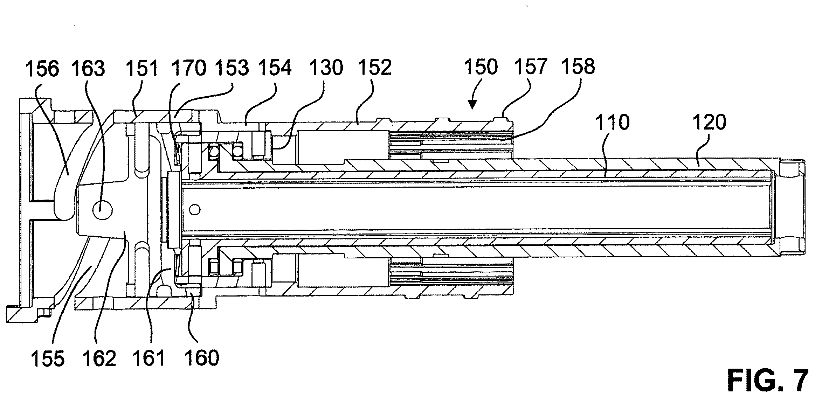

In der Fig. 7 ist dargestellt, wie die Einheit aus Führungshülse 110 und Lagerhalter 130 in einem hülsenförmigen Mechanikhalter 150 drehfest, aber in Längsrichtung verschiebbar gehalten ist.

Der Mechanikhalter 150 weist einen distalen Abschnitt 151 mit vergrössertem Innen- und Aussendurchmesser sowie einen proximalen Abschnitt 152 mit etwas geringerem Innen- und Aussendurchmesser auf. Diese beiden Abschnitte sind durch eine Stufe 153 verbunden. Die Aussenseite des distalen Abschnitts 151 ist starr in der Gehäusehülse 20 gehalten. Damit ist also auch der gesamte Mechanikhalter 150 gegenüber dem Gehäuse unbeweglich, bildet also funktional einen Teil des Gehäuses.

Angrenzend an die Stufe 153 sind im Mechanikhalter 150 mindestens zwei Längsschlitze 154 ausgebildet. Im Lagerhalter 130 sind Zapfen eingesetzt, die in der Fig. 7 nicht dargestellt sind. Diese ragen radial über den Lagerhalter 130 hinaus und in die Längsschlitze 154 des Mechanikhalters hinein. Hierdurch sind der Lagerhalter 130 und die mit diesem fest verbundene Führungshülse 110 in Längsrichtung zwischen einer distalen und einer proximalen Endstellung verschiebbar und drehgesichert im Mechanikhalter 150 geführt. Zum proximalen Ende hin ist auf der äusseren Mantelfläche des Mechanikhalters 150 ein Aussengewinde 157 ausgebildet. Auf der Innenfläche sind in diesem Bereich mehrere Längsnuten 158 ausgebildet.

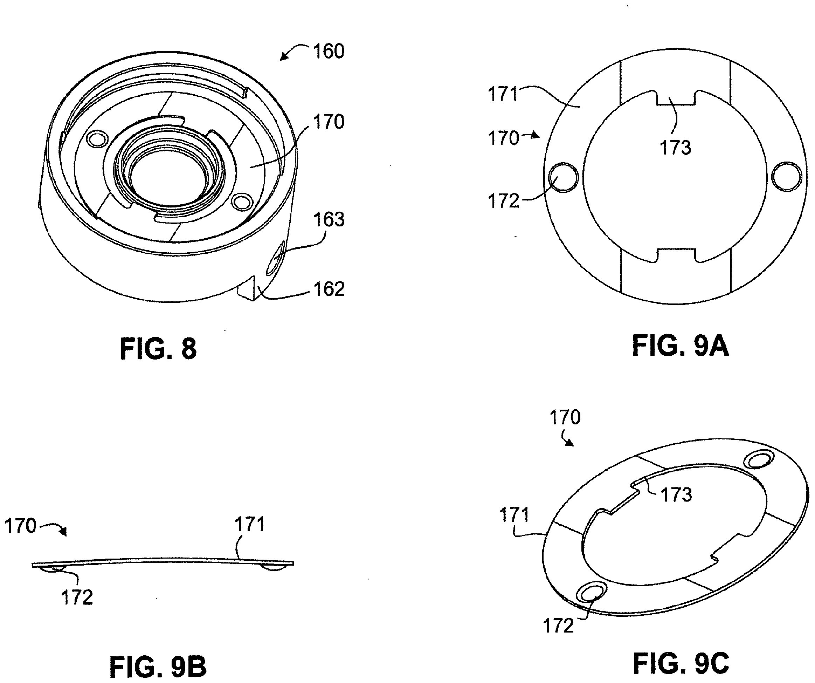

In distaler Richtung grenzt eine Bajonetthülse 160 an die Führungshülse 110 und den Lagerhalter 130 an, die auch in der Fig. 8 dargestellt ist. Sie ist auf dem Lagerhalter 130 in axialer Richtung gehalten und gegenüber diesem verdrehbar. Mit einem nach innen ragenden Ringflansch 161 stützt sie die Einheit aus Führungshülse 110 und Lagerhalter 130 mit darin gehaltener Kupplungshülse 120 in die distale Richtung ab. Die Bajonetthülse 160 weist zwei axial in die distale Richtung ragende und sich diametral gegenüberliegende Arme 162 auf, die radiale Öffnungen 163 aufweisen. In diese Öffnungen sind radial nach aussen ragende Zapfen eingesetzt, die in zwei als Kulissenführungen (Zwangsführungen) wirkenden Führungsschlitzen 155 des Mechanikhalters 150 laufen. Diese Führungsschlitze 155 sind so ausgestaltet, dass die Bajonetthülse 160 bei einer Drehung im Gegenuhrzeigersinn (im Sinne der oben angegebenen Definition, also bei Betrachtung entlang der Längsachse in distaler Richtung) zwangsweise auch axial in die proximale Richtung verschoben wird. Auf diese Weise wird die Einheit aus Führungshülse 110, Lagerhalter 130 und Kupplungshülse 120 in proximaler Richtung verschoben. Umgekehrt bewegt sich diese Einheit bei einer Drehung der Bajonetthülse 160 im Uhrzeigersinn in die distale Richtung. Parallel zu den Führungsschlitzen 155 verläuft ein weiteres Paar Führungsschlitze 156, um radiale Zapfen 36 eines Verriegelungsbereichs 35 der Karpulenhülse 30 aufzunehmen (vgl. Figuren 1 und 3). Beim Einführen der Karpulenhülse 30 in das Gehäuse unterliegt daher auch die Karpulenhülse einer Zwangsführung, so dass die Karpulenhülse 30 eine kombinierte Drehbewegung und Verschiebung ausführt. Die Karpulenhülse 30 ist dabei so ausgestaltet, dass sie bei ihrer Bewegung mit den Armen 162 der Bajonetthülse 160 gekoppelt wird und die Bajonetthülse 160 mitnimmt.

Die Führungsschlitze 155 sind von endlicher Länge und begrenzen die Bewegung der Bajonetthülse zwischen einer distalen und einer proximalen Endstellung. In der Fig. 7 ist die proximale Endstellung dargestellt. Insbesondere erlauben die Führungsschlitze 155 eine Drehung der Bajonetthülse um 90 Grad zwischen diesen Stellungen.

Um die Bajonetthülse in ihren beiden Endstellungen lösbar drehfest gegenüber der Führungshülse 110 und damit gegenüber der Gehäusehülse 20 zu fixieren, ist zwischen der Bajonetthülse 160 und der Führungshülse 110 eine Bajonettfeder 170 angeordnet. Diese ist in den Figuren 9A bis 9C im Detail dargestellt. Die Bajonettfeder 170 weist einen als Federelement wirkenden, im wesentlichen flachen und ringförmigen Grundkörper 171 auf. Aus diesem Grundkörper ragen zwei diametral gegenüberliegende, axial flach vorstehende Ausbuchtungen oder Vorsprünge 172 als Rastelemente oder Rastnocken axial in die distale Richtung vor. Zwei diametral gegenüberliegende flache Zungen 173 ragen nach innen und kommen in entsprechenden flachen Ausnehmungen der Führungshülse 110 zu liegen. Dadurch wird die Bajonettfeder 170 verdrehgesichert an der Führungshülse 110 gehalten. Wie aus der Fig. 9B erkennbar ist, ist der Grundkörper 171 leicht um eine Achse senkrecht zur Längsachse gebogen, und zwar in einer Weise, dass der Krümmungsmittelpunkt auf der selben Seite der Bajonettfeder wie die Vorsprünge 172 liegt (also distal). Hierdurch wird die Bajonettfeder 170 zwischen der Führungshülse 110 und der Bajonetthülse 160 permanent derart vorgespannt, dass die Vorsprünge 172 in distaler Richtung gegen die entsprechende Gegenfläche am Ringflansch 161 gedrückt werden. In dieser Gegenfläche sind vier Vertiefungen vorhanden, die in Abständen von 90 Grad um die Längsachse angeordnet sind. In der proximalen Endstellung kommen die Vorsprünge 172 in einem ersten Paar dieser Vertiefungen zu liegen, während sie in der distalen Endstellung, in der die Bajonetthülse um 90 Grad verdreht ist, in dem zweiten Paar der Vertiefungen aufgenommen werden. Dadurch gibt es also zwei definierte Raststellungen, in denen die Bajonetthülse 160 über die Bajonettfeder 170 mit den Vorsprüngen 172 lösbar mit der Führungshülse 110 verrastet. In beiden Stellungen muss zunächst eine gewisse Kraft überwunden werden, um die Bajonetthülse wieder in Richtung der jeweils anderen Endstellung zu bewegen. Die Vertiefungen können unterschiedlich ausgeprägt sein, z.B. unterschiedlich tief sein, so dass in den beiden Raststellungen eine unterschiedliche Lösekraft erforderlich ist.

In der einen Raststellung wird die Karpulenhülse 30 über ihre Kopplung mit der Bajonetthülse 160 dreh- und verschiebefest an der Führungshülse 110 und damit letztlich am Gehäuse gehalten. In der anderen Raststellung ist die Karpulenhülse 30 vom Gehäuse gelöst. Die Bajonetthülse 160 ist in dieser Stellung wiederum mit der Führungshülse 110 verrastet und dadurch am Gehäuse 20 dreh- und verschiebefest fixiert. Auf diese Weise ist sichergestellt, dass die Karpulenhülse bei Einschieben in die Führungsschlitze 156 die Arme 162 der Bajonetthülse wieder in der korrekten Stellung um die Längsachse "findet" und diese dann nach dem Lösen der Rastverbindung mitnehmen kann.

Im vorliegenden Beispiel sind die Rastelemente als Vorsprünge 172 einstückig mit dem Federelement in Form des Grundkörpers 171 ausgebildet. Stattdessen kann aber auch ein getrenntes Rastelement, z.B. in Form eines starren Rings mit Rastnocken, vorgesehen sein, das vom Federelement in die axiale Richtung gedrückt wird. Das Rastelement kann statt Vorsprüngen auch Vertiefungen aufweisen, die dann mit entsprechenden Vorsprüngen der Gegenfläche zusammenwirken. Im vorliegenden Beispiel ist das Rastelement drehfest zum Gehäuse. Stattdessen kann es auch drehfest zur Bajonetthülse sein. Das Federelement kann zur Erzeugung einer axialen Kraft auch anders ausgestaltet sein. Es sind also vielfache Abwandlungen der hier dargestellten Verriegelung zwischen der Karpulenhülse 30 und dem Gehäuse möglich.

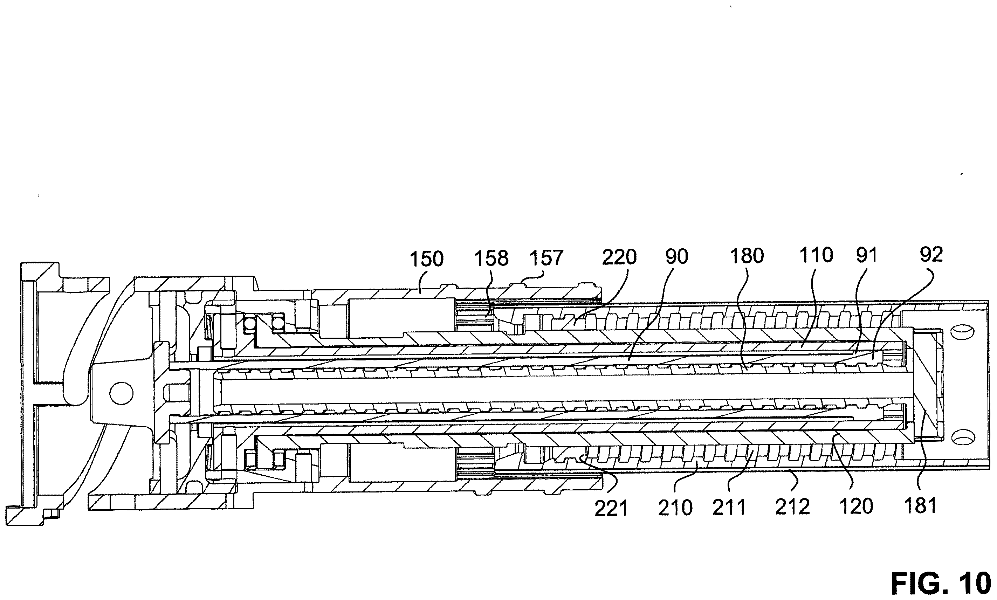

In der Fig. 10 sind die in der Fig. 7 dargestellten Teile der Injektionsvorrichtung zusammen mit weiteren Bauteilen dargestellt. Insbesondere ist nun die Vorschubhülse 90 in die Führungshülse 110 eingeschoben. An ihrem proximalen Ende weist die Vorschubhülse 90 ein kurzes Innengewinde 92 auf, in welchem eine hohle Aussengewindestange 180 geführt ist. Diese ist an ihrem proximalen Ende über einen Querbolzen 181 starr mit der Kupplungshülse 120 verbunden. Ein Vorschub der Vorschubhülse 90 in distaler Richtung erfolgt also, indem die Kupplungshülse 120, die ja drehbar gelagert ist, eine Drehbewegung ausführt. Diese Drehbewegung bewirkt wegen der starren Verbindung zwischen Kupplungshülse 120 und Aussengewindestange 180 eine Drehung auch der Aussengewindestange 180. Die Vorschubhülse 90 läuft mit ihrem Innengewinde 92 auf der Aussengewindestange 180, ähnlich einer Mutter. Die Vorschubhülse 90 ist drehfest gegenüber der Führungshülse 110, da sie über die Führungsnocken 91 in Längsrillen auf der Innenseite der Führungshülse 110 läuft. Auf diese Weise wird die Vorschubhülse 90 bei einer Drehung der Aussengewindestange 180 axial vorgeschoben. Insgesamt wird also eine Drehbewegung der Kupplungshülse 120 in eine axiale Verschiebung der Vorschubhülse 90 umgewandelt.

Wie aus der Fig. 3 erkennbar ist, wird die Vorschubhülse 90 bei dieser Vorschubbewegung durch eine lange, auf Druck belastete Schraubenfeder 190 unterstützt, die im Inneren der Gewindestange 180 angeordnet und auf einer Führungsnadel 200 geführt ist. Die Schraubenfeder 190 drückt eine ringförmige Verdickung 201 nahe dem distalen Ende der Führungsnadel 200 in die distale Richtung gegen den Vorschubflansch 100. Ein axialer Zapfen 202 ragt in ein entsprechendes Sackloch des Vorschubflansches 100 und ist in diesem Sackloch drehbar.

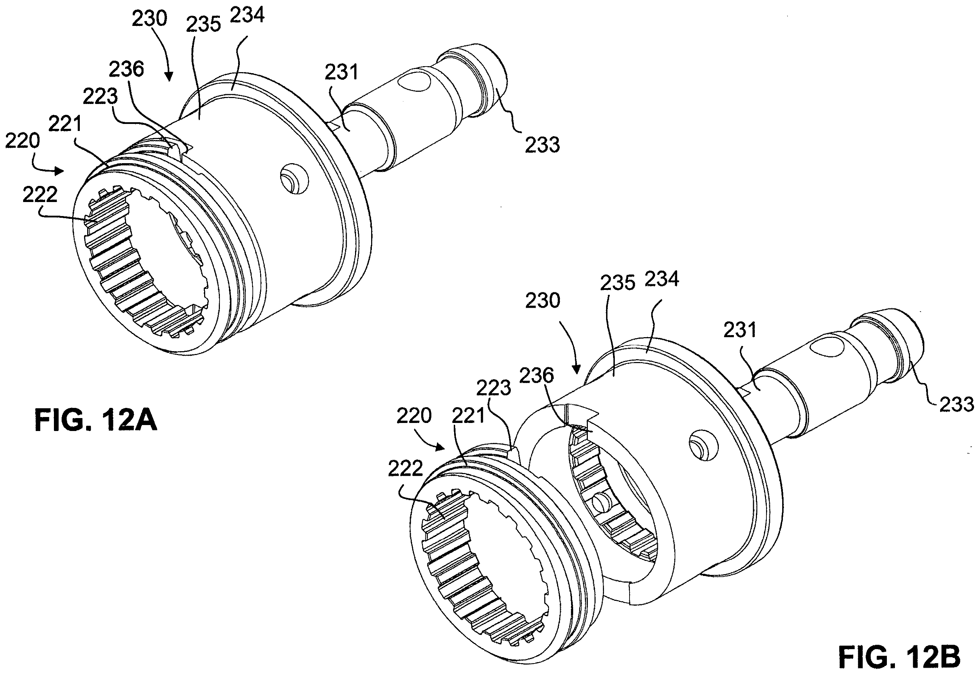

Des weiteren ist in der Fig. 10 in den Mechanikhalter 150 von der proximalen Seite her eine im Wesentlichen zylindrische Übertragungshülse 210 eingeschoben, welche die Kupplungshülse 120 teilweise umgibt. Die Übertragungshülse 210 weist auf der Aussenseite eine Mehrzahl von Längsrippen 212 auf. Der Aussendurchmesser der Übertragungshülse 210 ist dabei so gewählt, dass diese trotz ihrer äusseren Längsrippen frei im Inneren des Mechanikhalters 150 drehbar ist. Auf der Innenseite weist die Übertragungshülse 210 ein Innengewinde 211 auf, in dem ein Dosisbegrenzungsring 220 mit einem entsprechenden Aussengewinde 221 läuft. Im Inneren des Dosisbegrenzungsrings 220 sind in den Figuren 12A und 12B besonders gut erkennbare Längsnuten 222 vorhanden, in welche die Längsrippen 122 der Kupplungshülse 120 (vgl. Fig. 6) eingreifen. Dadurch ist der Dosisbegrenzungsring 220 einerseits drehfest in axialer Richtung auf der Kupplungshülse verschiebbar, andererseits im Innengewinde der Übertragungshülse 210 geführt. Eine Drehung der Kupplungshülse 120 gegenüber der Übertragungshülse 210 führt also zu einer Drehung und axialen Verschiebung des Dosisbegrenzungsrings 220.

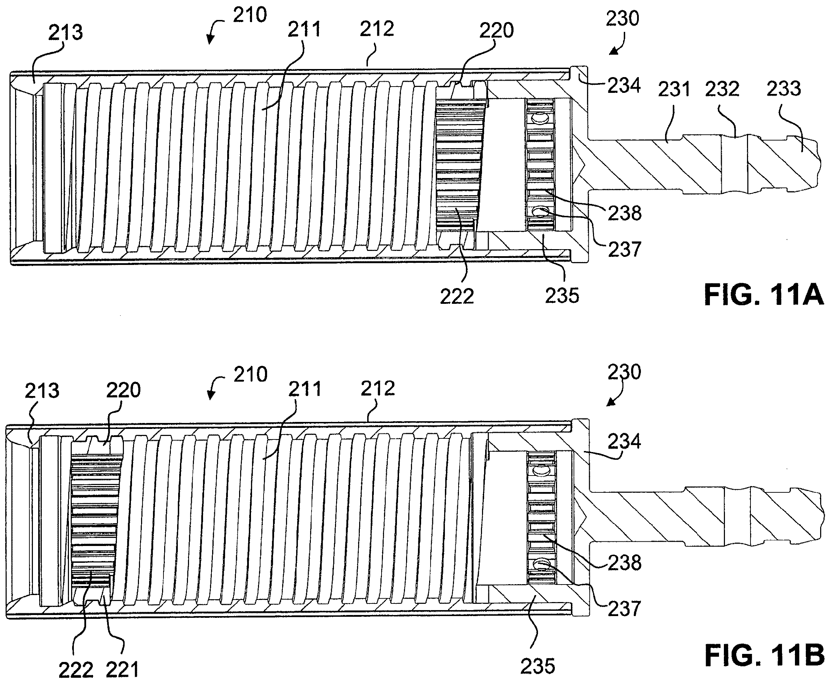

Der axiale Verschiebungsbereich des Dosisbegrenzungsrings ist in distaler wie in proximaler Richtung begrenzt. Dies soll anhand der Figuren 11A, 11B, 12A und 12B erläutert werden.

In den Figuren 11A und 11B ist eine Kupplungswelle 230 mit der Übertragungshülse 210 verbunden. Die Kupplungswelle weist eine Achse 231 mit einer Querbohrung 232 nahe dem proximalen Ende 233 auf. Vom distalen Ende der Achse aus erstreckt sich ein umlaufender Flansch 234 radial nach aussen. Von diesem aus erstreckt sich wiederum ein Ringflansch 235 axial in distaler Richtung. Der Aussendurchmesser des Flansches 234 ist grösser als derjenige des Ringflansches 235, wodurch der Flansch 234 radial über den Ringflansch 235 übersteht und einen Anschlag für die Übertragungshülse 210 bildet. Der Ringflansch 235 ist in die Übertragungshülse 210 eingeschoben, so dass diese mit ihrem proximalen Ende am Flansch 234 ansteht. Der Ringflansch ist über radiale Stifte in der Übertragungshülse 210 gesichert, die in Bohrungen 237 eingeschoben sind. Dadurch sind die Kupplungswelle 230 und die Übertragungshülse 210 drehfest und verschiebegesichert miteinander verbunden. In der Innenfläche des Ringflansches 235 sind mehrere Längsnuten 238 ausgebildet.

Die Figuren 12A und 12B zeigen die Kupplungswelle 230 und den Dosisbegrenzungsring 220 alleine. Am Dosisbegrenzungsring 220 ist ein Radialanschlag 223 ausgebildet, der mit einem entsprechenden Radialanschlag 236 am Ringflansch 235 der Kupplungswelle zusammenwirkt. Unter einem Radialanschlag wird eine Anschlagfläche verstanden, deren Flächennormale im Wesentlichen in die tangentiale Richtung weist, und die dazu ausgebildet ist, mit einer entsprechenden Gegenfläche zusammenzuwirken. Der Radialanschlag wird also primär in tangentialer Richtung (also in Drehrichtung) beansprucht statt in axialer Richtung. Dadurch vermeidet ein Radialanschlag die Gefahr des Verklemmens, die besteht, wenn zwei Teile über eine Schraubenverbindung axial aufeinanderstossen, und die zumal bei geringer Steigung des Schraubengewindes besonders ausgeprägt ist. Der Radialanschlag 236 begrenzt hier die Schraubenbewegung des Dosisbegrenzungsrings 220 in proximaler Richtung. In der Fig. 11A ist der Dosisbegrenzungsring 220 in der resultierenden proximalen Endstellung, in der Fig. 11B dagegen in einer distalen Ausgangsstellung gezeigt.

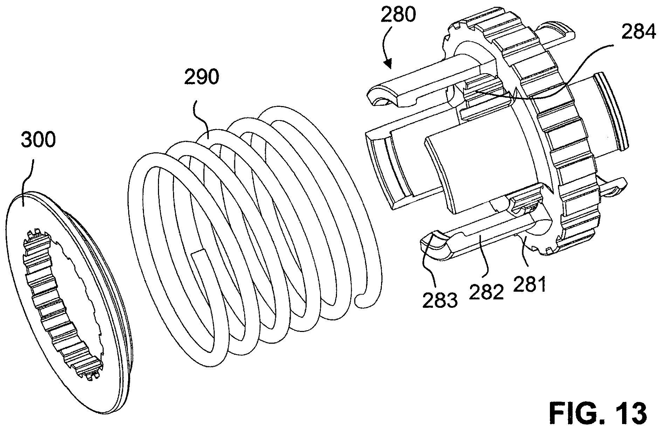

In einen nach innen weisenden, in die distale Richtung abgeschrägten Ringflansch 213 am distalen Ende der Übertragungshülse 210 ist das proximale Ende einer Arretierhülse 280 drehbar eingeklickt. Der besseren Übersichtlichkeit wegen ist die Arretierhülse 280 in der Fig. 10 nicht dargestellt. Sie ist aber in der Fig. 13 gezeigt. Die Arretierhülse umfasst einen ringförmigen Hauptkörper 281, von dem sich vier sich in die distale Richtung erstreckende Arme 282 in die distale Richtung erstrecken. Der Hauptkörper weist an seiner Innenfläche Längsnuten 284 auf, die mit den Längsrippen 122 der Kupplungshülse 120 verzahnt sind. Dadurch ist die Arretierhülse 280 in Längsrichtung relativ zur Kupplungshülse 120 verschiebbar, aber gegenüber dieser verdrehgesichert. Am Ende der Arme 282 sind sich nach innen erstreckende Flanschbereiche 283 vorhanden. Der mögliche Verschiebungsbereich wird in proximaler Richtung durch diese Flanschbereiche begrenzt. Diese stossen in der proximalen Endstellung der Arretierhülse 280, wie sie in der Fig. 3 dargestellt ist, an das distale Ende der Längsrippen 122 der Kupplungshülse 120 an. Auf der äusseren Umfangsfläche des Hauptkörpers 281 sind Längsrippen ausgebildet. Diese Längsrippen greifen in der Stellung der Fig. 3 in die inneren Längsnuten 158 des Mechanikhalters 150 ein. Dadurch ist die Arretierhülse 280 in dieser Stellung axial gegenüber dem Mechanikhalter 150 verschiebbar, aber verdrehgesichert. Letztlich sichert die Arretierhülse 280 in dieser Stellung also die Kupplungshülse 120 gegen eine Drehung im Mechanikhalter 150. Wie weiter unten noch erläutert wird, ist die Arretierhülse 280 jedoch so weit in der distalen Richtung verschiebbar, dass sie ausser Eingriff mit dem Mechanikhalter 150 kommen kann und gegenüber diesem dann zusammen mit der Kupplungshülse 120 drehbar ist.

Die Arretierhülse 280 ist mittels einer Kupplungsfeder 290 in die proximale Richtung vorgespannt. Die Kupplungsfeder hat die Form einer auf Druck belasteten Schraubenfeder, welche die Arme 282 der Arretierhülse 280 umgibt und mit ihrem proximalen Ende an der distalen Stirnseite des Hauptkörpers 281 anliegt. Am distalen Ende der Kupplungsfeder 290 ist diese auf einem Stützring 300 gehalten, der in der distalen Richtung an den Lagerhalter 130 anstösst und an dessen Innenseite Längsnuten ausgebildet sind.

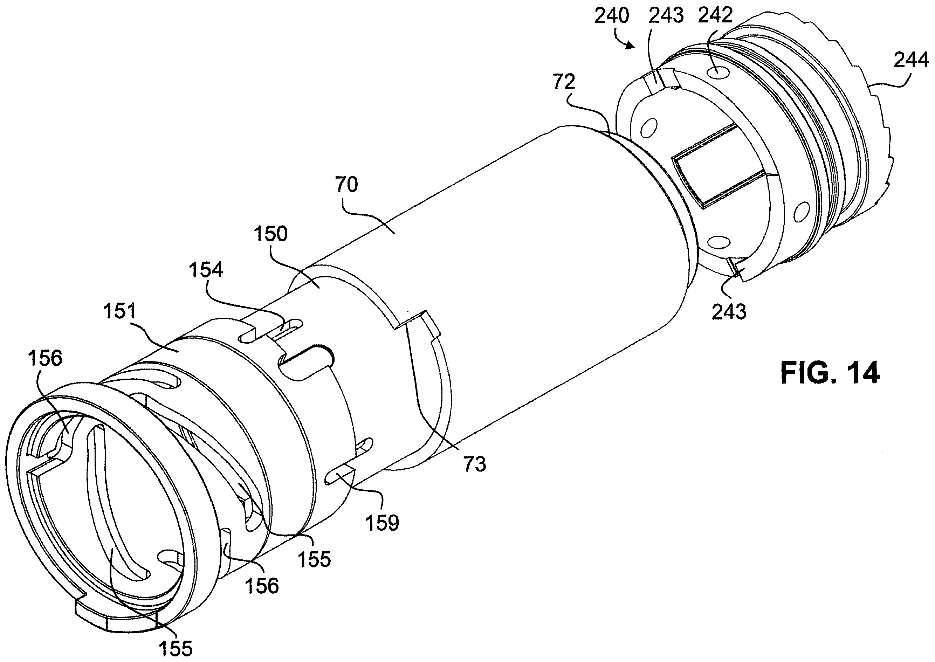

In der Fig. 14 ist nun die Einheit der Fig. 10 mit nochmals weiteren Bauteilen dargestellt. Auf dem Mechanikhalter 150 ist nun die schon zuvor erwähnte Anzeigetrommel 70 gehalten. Ausserdem ist eine Anschlaghülse 240 erkennbar. Wie in der Fig. 2 zu erkennen ist, ist die Anschlaghülse 240 durch Stifte, die in die in der Fig. 14 erkennbaren radialen Löcher 242 ragen, unbeweglich mit der Gehäusehülse 20 verbunden. An ihrem distalen Ende sind Radialanschläge 243 ausgebildet. Ihr proximales Ende ist gezackt ausgebildet, d.h. es sind stirnseitig Zähne 244 für eine noch zu beschreibende Ratschenverbindung ausgebildet.

Die Anzeigetrommel 70 weist ein Innengewinde auf, welches in der Fig. 3 erkennbar ist. Dieses läuft auf dem Aussengewinde 157 des Mechanikhalters, welches besonders gut in den Figuren 7 und 10 erkennbar ist. An ihrem proximalen Ende verjüngt sich die Anzeigetrommel 70 zu einem ringförmigen Bereich 72. An der Innenseite des ringförmigen Bereichs 72 sind Längsnuten ausgebildet. Mit diesen Längsnuten ist die Anzeigetrommel 70 verdrehgesichert, aber in Längsrichtung verschiebbar auf den Längsrippen 212 der Übertragungshülse 210 geführt. Durch die Kombination dieser Längsführung auf der Übertragungshülse und der Gewindeführung auf dem Mechanikhalter führt eine Drehung der Übertragungshülse 210 zu einer kombinierten Drehung und Längsverschiebung der Anzeigetrommel 70. Diese Bewegung ist durch Radialanschläge in beide Richtungen begrenzt. Am proximalen Ende wirkt ein Radialanschlag mit dem Radialanschlag 243 der Anschlaghülse 240 zusammen. Am distalen Ende wirkt ein entsprechender Radialanschlag 73 mit einem Radialanschlag 159 des Mechanikhalters 150 zusammen. Dadurch ist die Schraubenbewegung der Anzeigetrommel 70 in beide Richtungen durch Radialanschläge limitiert.

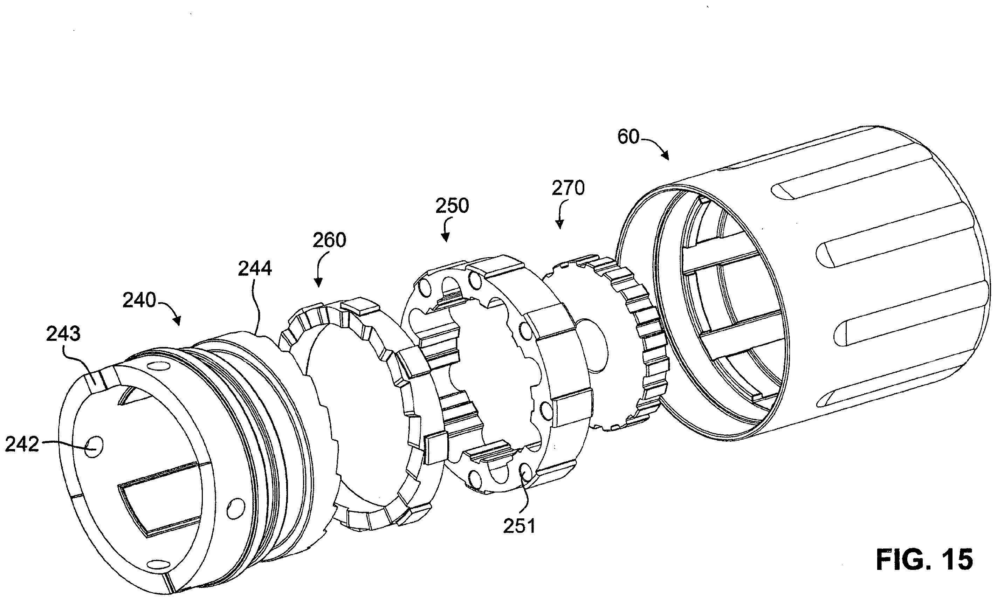

Im Folgenden wird nun der Mechanismus zum Einstellen einer Dosis und zum Auslösen der Verabreichung dieser Dosis anhand der Figuren 3 und 15 erläutert. Am proximalen Ende der Gehäusehülse 20 ist die schon erwähnte Dosierhülse 60 angeordnet. Sie ist mit einem Federring 61 axial verschiebegesichert und drehbar an der Anschlaghülse 240 fixiert. Die Dosierhülse 60 ist über eine Rutschkupplung in Form einer Ratschenverbindung sowohl im Uhrzeigersinn als auch gegen den Uhrzeigersinn um die Längsachse gegen die Gehäusehülse 20 verdrehbar, wobei sie mehrere vordefinierte Raststellungen einnehmen kann. Dieser Mechanismus soll im Folgenden erläutert werden.

Im Inneren der Dosierhülse 60 ist ein Innenring 250 angeordnet und starr mit der Dosierhülse 60 verbunden. Der Innenring 250 weist in seiner radialen Innenfläche eine Mehrzahl von Längsnuten auf. In distaler Richtung vom Innenring 250 ist ein Ratschenring 260 axial verschiebbar, aber drehgesichert in der Dosierhülse 60 gehalten. Der Ratschenring 260 ist auf seiner distalen Stirnseite gezackt ausgebildet, und zwar komplementär zu den Zähnen 244 der gezackten proximalen Stirnseite der Anschlaghülse 240, so dass Zähne des Ratschenrings 260 in Vertiefungen der Stirnseite der Anschlaghülse 240 eingreifen können und umgekehrt. Der Ratschenring 260 ist zwischen der distalen Stirnfläche des Innenrings 250 und der gezackten proximalen Stirnseite der Anschlaghülse 240 um einen gewissen Betrag axial verschiebbar. Der Betrag, um den eine axiale Verschiebung möglich ist, ist dabei mindestens so gross, dass die gezackten Stirnseiten des Ratschenrings 260 und der Anschlaghülse 240 ausser Eingriff gelangen können. Der Ratschenring 260 wird durch eine Federkraft elastisch gegen die Anschlaghülse 240 gedrückt. Hierzu sind im Innenring 250 mehrere axiale Bohrungen 251 in Form von Sacklöchern vorhanden. In mindestens einer dieser Bohrungen, bevorzugt in mindestens zwei Bohrungen in gleichmässigem Abstand entlang des Umfangs des Rings, sind auf Druck belastete Schraubenfedern 252 eingesetzt. Die Schraubenfedern 252 pressen den Ratschenring elastisch gegen die Anschlaghülse.

In der Ruhestellung befindet sich der Ratschenring 260 mit seiner gezackten Stirnseite im Eingriff mit der gezackten Stirnseite der Anschlaghülse 240. Dadurch nehmen der Ratschenring und die mit ihm verbundene Dosierhülse 60 eine von mehreren definierten Winkelstellungen um die Längsachse ein. Bei einer Drehung der Dosierhülse 60 relativ zur Gehäusehülse 20 gleiten die Zähne des Ratschenrings 260 und der Anschlaghülse 240 gegen die axiale Federkraft der Schraubenfedern 252 aufeinander, bis sie ausser Eingriff gelangen und in der nächsten definierten Winkelstellung wieder in Eingriff gelangen. Auf diese Weise entsteht eine durch Drehen mit einem genügenden Drehmoment federnd lösbare Rastverbindung in mehreren vordefinierten Winkelstellungen der Dosierhülse 60 relativ zur Gehäusehülse 20. Dieser Mechanismus kann auch als Doppelrutschkupplung bezeichnet werden.

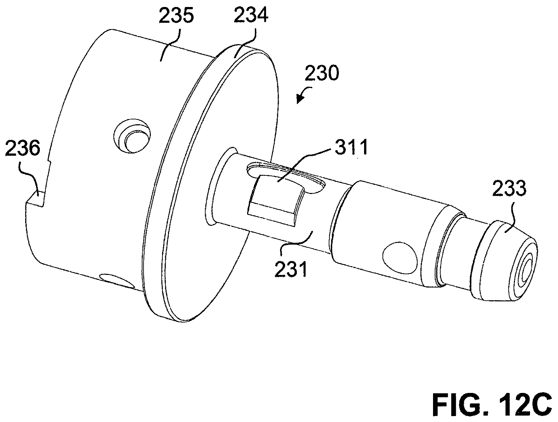

Durch Drehung der Dosierhülse 60 im Uhrzeigersinn lässt sich die Spiralfeder 310 spannen, die in der Fig. 3 angedeutet ist. Die Spiralfeder 310 weist eine Mehrzahl von Federwindungen auf, die um die Längsachse umlaufen und radial zur Längsachse übereinander angeordnet sind. Das innere Ende der Spiralfeder 310 ist an einem in der Fig. 12C besonders gut erkennbaren Federhaltebereich 311 der Kupplungswelle 230 befestigt. Das äussere Ende der Spiralfeder 310 ist an einer Federhülse 320 angebracht, welche drehfest in der Anschlaghülse 240 gehalten ist.

Auf der Kupplungswelle 230 ist eine Kupplungsscheibe 270 montiert und über einen Stift 271 in der Querbohrung 232 der Kupplungswelle 230 gegen Drehung und Verschiebung gesichert. Die Kupplungsscheibe 270 weist an ihrer äusseren Umfangsfläche eine Mehrzahl von Längsrippen auf. In der Stellung der Fig. 3 greifen diese Längsrippen in die dazu komplementären Längsnuten an der Innenseite des Innenrings 250 ein, können aber durch eine axiale Verschiebung ausser Eingriff gebracht werden.

Die Dosierhülse 60 weist eine axiale Durchgangsöffnung auf, in welcher der Druckknopf 80 axial verschiebbar angeordnet ist. Der Druckknopf 80 ist mit einer Mehrzahl von radial federnden Armen 81 drehbar und verschiebegesichert auf dem proximalen Ende 233 der Kupplungswelle 230 aufgeklickt. Er stösst mit seinem distalen Ende gegen eine proximale Stirnfläche der Kupplungsscheibe 270. Im Inneren des Druckknopfs 80 befindet sich eine Schraubenfeder 82, welche mit ihrem proximalen Ende an der inneren Stirnfläche des Druckknopfs anliegt und mit ihrem distalen Ende gegen einen Auflagering 83 drückt. Der Auflagering 83 weist auf seiner äusseren Umfangsfläche Längsrippen auf, die in entsprechenden Längsnuten in der inneren Mantelfäche des Druckknopfes 80 geführt sind. Dadurch ist der Auflagering 83 im Druckknopf 80 drehfest und axial verschiebbar angeordnet. An seiner distalen Stirnseite ist der Auflagering 83 flach gezackt ausgebildet. Die proximale Stirnseite der Kupplungsscheibe 270 ist hierzu komplementär gezackt ausgebildet, so dass der Auflagering 83 mit der Kupplungsscheibe 270 axial verzahnt ist. Beim Ausschütten des Medikaments dreht sich die Kupplungsscheibe 270 gegenüber dem Auflagering 83. Dabei gleiten die gezackten Flächen aufeinander, so dass die Verzahnung abwechselnd in und ausser Eingriff gerät. Dadurch wird ein charakteristisches Klickgeräusch erzeugt, das dem Benutzer anzeigt, dass gerade eine Verabreichung stattfindet. Die Verzahnung von Auflagering 83 und Kupplungsscheibe 270 ist dabei vorzugsweise so ausgestaltet, dass jedes Klicken gerade einer Einheit (oder eines vorbestimmten Vielfachen einer Einheit) des verabreichten Medikaments entspricht.

### Abdichtung

Die gesamte Mechanik für die Einstellung der Dosis und die Ausschüttung ist spritzwassergeschützt in der Gehäusehülse 20 untergebracht. Hierzu sind insgesamt vier Dichtungen D1, D2, D3 und D4 vorhanden. Die Dichtung D1 umfasst einen Dichtring, der zwischen dem Mechanikhalter 150 und der Bajonetthülse 160 dichtend anliegt. Der Mechanikhalter 150 ist unbeweglich und dicht im Gehäuse 2 montiert, während die Bajonetthülse 160 gegenüber dem Mechanikhalter 160 sowohl verschiebbar als auch drehbar ist und dabei über die Dichtung D1 gegenüber dem Gehäuse gedichtet ist. Die Dichtung D2 umfasst einen weiteren, hier flach ausgebildeten Dichtring, der dichtend zwischen der Bajonetthülse 160 und der glatten Aussenseite der Vorschubhülse 90 anliegt. Des weiteren ist der Vorschubflansch 100 dicht auf der Vorschubhülse 90 angeordnet. Damit ist der proximal von der Bajonetthülse 160 liegende Bereich der Injektionsvorrichtung, einschliesslich dem Inneren der Vorschubhülse 90, komplett gegen den distal hiervon liegenden Bereich gedichtet. Falls Flüssigkeiten in diesen distalen Bereich gelangen sollten, z.B. wegen eines Bruchs der Medikamentenkarpule 40, so können diese nicht in die diffizile Mechanik eindringen und diese verschmutzen oder verkleben.

Die anderen beiden Dichtungen befinden sich am proximalen Ende des Injektionsgeräts. Die Dichtung D3 umfasst einen Dichtring, der zwischen der Dosierhülse 60 und der Anschlaghülse 240 dichtend anliegt. Die Anschlaghülse 240 ist unbeweglich und dicht in der Gehäusehülse 20 montiert, während die Dosierhülse 60 gegenüber der Anschlaghülse 240 verdrehbar ist. Die Dichtung D4 umfasst einen weiteren Dichtring, der dichtend zwischen der Dosierhülse 60 und dem Druckknopf 80 anliegt. Zudem ist auf dem Fenster 21 eine transparente Fensterabdeckung 22 flüssigkeitsdicht aufgesetzt. Hierdurch ist die gesamte Mechanik, die nach aussen hin durch die Gehäusehülse 20, die Dosierhülse 60 und den Druckknopf 80 begrenzt wird, auch nach aussen hin komplett gedichtet und so vollständig gegen das Eindringen von Flüssigkeiten geschützt. Auch ein Regenguss oder ein versehentlich vom Benutzer umgeworfenes Wasserglas können dem Injektionsgerät also nichts anhaben.

Insbesondere die Dichtung zur Vorschubhülse hin ist bevorzugt derart ausgebildet, dass sie als Abstreifer wirkt, ähnlich einem Scheibenwischer beim Auto. Hierzu besteht mindestens zur distalen Seite hin vorzugsweise ein möglichst kleiner Kontaktwinkel zwischen den Oberflächen des Dichtelements und der Vorschubhülse. Dieser kann unterhalb von 90 Grad liegen.

Anstelle von konventionellen Dichtungen oder zusätzlich hierzu können die gegeneinander zu dichtenden Teile eine hydrophobe Oberfläche aufweisen, insbesondere aus einem hydrophoben Material ausgebildet oder damit beschichtet sein. Eine hydrophobe Oberfläche verhindert, dass die Teile benetzt werden. Dadurch perlen Wassertropfen ab, und es wird ein Kriechen von Flüssigkeiten durch Spalte zwischen den zu dichtenden Teilen aufgrund von Kapillareffekten wirksam verhindert. Die mit einer hydrophoben Oberfläche versehenen, gegeneinander zu dichtenden Teile können also in einem gewissen Abstand (Spalt) zueinander angeordnet sein, ohne dass die Dichtwirkung verloren geht ("virtuelle Dichtung").

Unter einer hydrophoben Oberfläche wird hier eine Oberfläche verstanden, für die der Kontaktwinkel eines Wassertropfens mindestens 90 Grad beträgt, bevorzugt mindestens 110 Grad. Der Kontaktwinkel ist der Winkel zwischen den Flächennormalen des Wassertropfens und der betreffenden Oberfläche an der Kontaktstelle. Beispiele für Materialien mit hydrophoben Eigenschaften sind PTFE (Polytetrafluorethylen) oder PVDF (Polyvinylidenfluorid). Viele weitere Materialien, insbesondere auch für dünne Beschichtungen im Bereich von wenigen Mikrometern, sind bekannt.

In Versuchen wurden verschiedene Stifte sowie eine Hülse nanotechnologisch mit einer hydrophoben Beschichtung versehen. Es wurden 20 Stifte mit Aussendurchmessern von 10.0 bis 11.9 mm in Abstufungen von 0.1 mm untersucht. Die Stifte wurden zentral in einer Hülse mit 12 mm Innendurchmesser angeordnet, was Spaltdicken von 0.05 mm bis 1.0 mm in Abstufungen von 0.05 mm entspricht. Das Innere der Hülse wurde dann mit Wasser beaufschlagt. Es wurde eine Dichtwirkung bis zu einer Spaltdicke von ca. 0.5 mm beobachtet. Bei gegenseitiger Drehung zwischen Stift und Hülse wurde eine Dichtwirkung bis zu einer Spaltdicke von ca. 0.25 mm beobachtet.

Um die Dichtwirkung zu verbessern, können die Oberflächen mikro- oder nanostrukturiert werden, d.h. mit Strukturen versehen werden, deren Abmessungen im Nanometer- bis Mikrometerbereich liegen. Diese Strukturen können eine Vorzugsrichtung aufweisen, um das Fliessen von Flüssigkeiten auf der Oberfläche in eine Richtung zu hemmen. So können z.B. Schuppen vorgesehen werden.

### Kupplungen

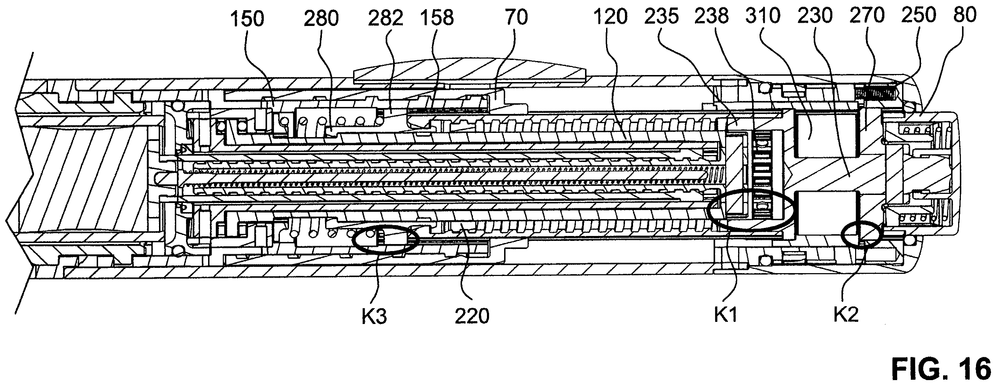

In Folgenden soll nun die Funktionsweise des Injektionsgeräts erläutert werden. Hierzu wird zunächst auf die Fig. 16 verwiesen, in welcher das Injektionsgerät in seiner Ausgangsstellung vor dem ersten Gebrauch dargestellt ist. Die oben erläuterte Mechanik zum Einstellen und Ausschütten einer Dosis verfügt über drei Kupplungen K1, K2, und K3 für die Übertragung von Drehmomenten. Jede dieser Kupplungen kann durch eine axiale Bewegung zweier Bauteile gegeneinander in und ausser Eingriff gebracht werden.

Die Kupplung K1 wird durch die Längsnuten auf der Innenfläche des axialen Flansches 235 der Kupplungswelle 230 als Kupplungseingangsglied im Zusammenspiel mit den Längsrippen 122 auf der Aussenseite der Kupplungshülse 120 (vgl. Fig. 5) als Kupplungsausgangsglied gebildet. In der Stellung der Fig. 16 ist diese Kupplung ausgekuppelt, d.h., die durch die Längsnuten und Längsrippen gebildete Verzahnung ist ausser Eingriff. Die Kupplung K1 lässt sich durch eine axiale Verschiebung der Kupplungswelle 230 in distaler Richtung einkuppeln.

Die Kupplung K2 wird durch die Längsnuten in der radialen Innenfläche des Innenrings 250 als Kupplungseingangsglied im Zusammenspiel mit den Längsrippen auf der radialen Aussenfläche der Kupplungsscheibe 270 als Kupplungsausgangsglied gebildet. In der Stellung der Fig. 16 ist diese Kupplung eingekuppelt, d.h., die durch die Längsnuten und Längsrippen gebildete Verzahnung ist im Eingriff. Die Kupplung K2 lässt sich durch eine axiale Verschiebung der Kupplungsscheibe 270 in distaler Richtung auskuppeln.

Die Kupplung K3 wird durch die Längsrippen auf der Aussenseite des äusseren Ringflansches 282 der Arretierhülse 280 als Kupplungseingangsglied im Zusammenspiel mit den Längsnuten 158 auf der Innenfläche des Mechanikhalters 150 als Kupplungsausgangsglied gebildet. In der Stellung der Fig. 13 ist diese Kupplung eingekuppelt. Sie lässt sich durch eine axiale Verschiebung der Arretierhülse 280 in distaler Richtung auskuppeln.

Alle drei Kupplungen K1, K2 und K3 lassen sich ein- bzw. auskuppeln, indem der Druckknopf 80 genügend weit axial verschoben wird. Dabei wird eine bestimmte Reihenfolge eingehalten. Beim Eindrücken des Druckknopfes 80 werden die Kupplungsscheibe 270 und die mit ihr fest verbundene Kupplungswelle 230 in distaler Richtung verschoben. Dabei kommt zunächst die Kupplung K1 in Eingriff, d.h., die Kupplungswelle wird zur Drehmomentübertragung mit der Kupplungshülse 120 gekoppelt. Gleichzeitig schiebt die Kupplungswelle 230 die Übertragungshülse 210 in die distale Richtung vor. Diese nimmt die Arretierhülse 280 in distaler Richtung mit, wodurch die Kupplungsfeder 290 komprimiert wird. Wenn die Kupplung K1 erstmals in Eingriff gelangt, ist die Arretierhülse 280 noch nicht genügend weit vorgeschoben, um mit ihrem äusseren Ringflansch 282 ausser Eingriff mit dem Mechanikhalter 250 zu gelangen. Die Kupplung K3 ist also zunächst weiterhin eingekuppelt. Dasselbe gilt für die Kupplung K2: Die Kupplungsscheibe 270 ist zunächst noch immer im Eingriff mit dem Innenring 250. Zunächst sind nun also alle drei Kupplungen eingekuppelt. Wenn der Druckknopf 80 weiter eingeschoben wird, gerät als nächstes die Kupplung K2 ausser Eingriff. Bei einem noch weiteren Einschieben gerät schliesslich auch die Kupplung K3 ausser Eingriff. Es wird also die folgende Reihenfolge eingehalten:
- Ausgangszustand: K1 ausgekuppelt, K2 und K3 eingekuppelt.
- Eindrücken des Druckknopfs 80: K1 kuppelt ein, danach kuppelt K2 aus, danach kuppelt K3 aus.

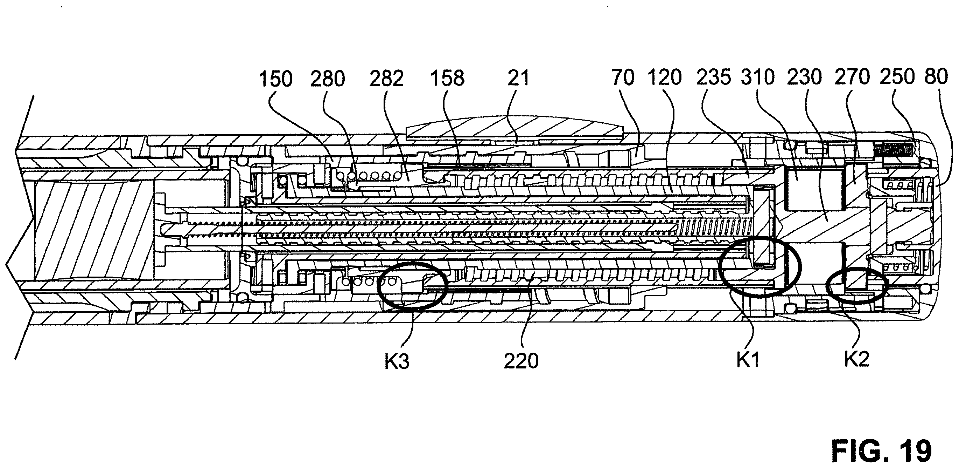

Die später noch näher beschriebene Fig. 19 zeigt das Injektionsgerät bei vollständig eingedrücktem Druckknopf 80. Die Kupplung K1 ist nun eingekuppelt, während die Kupplungen K2 und K3 ausgekuppelt sind.

Bei Loslassen des Druckknopfs 80 läuft das Ineinandergreifen der Kupplungen in umgekehrter Reihenfolge ab. Dabei drückt die Kupplungsfeder 290 die Arretierhülse 280, die Übertragungshülse 210, die Kupplungswelle 230, die Kupplungsscheibe 270 und den Druckknopf 80 in die distale Ausgangsstellung zurück.

Die Kupplungen K1, K2 und K3 sowie die Ratschenverbindung ermöglichen die gezielte Übertragung von Drehmomenten zwischen fünf funktional selbstständigen Einheiten. Eine erste Einheit wird durch die Gehäusehülse 20, den Mechanikhalter 150, die Anschlaghülse 240 und den Federring 320 gebildet. Diese Einheit kann funktional als Halteeinrichtung oder als ein Gehäuse in einem erweiterten Sinn aufgefasst werden. Sie stellt das stationäre Bezugssystem für alle Bewegungen dar.

Eine zweite Einheit wird durch die Dosierhülse 60, den Innenring 250 und den Ratschenring 260 gebildet. Sie kann funktional als drehbare Dosiereinrichtung aufgefasst werden. Diese Dosiereinrichtung wird über die Ratschenverbindung am Gehäuse lösbar, aber bis zu einem gewissen Wert drehmomentenfest gehalten.

Eine dritte Einheit wird durch die Kupplungsscheibe 270, die Kupplungswelle 230 und die Übertragungshülse 210 gebildet, die ja alle starr miteinander verbunden sind, sowie durch die damit verbundene Spiralfeder 310, die als das eigentliche Antriebselement wirkt. Diese Einheit kann als Antriebseinrichtung aufgefasst werden. Die Drehbewegung der Antriebseinrichtung ist durch zwei Begrenzungselemente limitiert, die beide an der Übertragungseinrichtung geführt sind. Das erste Begrenzungselement wird durch die Anzeigetrommel 70 gebildet, welche den Bewegungsbereich der Antriebseinrichtung in beide Richtungen, einer Dosierrichtung und einer Korrektur- und Ausschüttrichtung, limitiert. Das zweite Begrenzungselement wird durch den Dosisbegrenzungsring 220 gebildet, welcher den Bewegungsbereich der Antriebsseinrichtung zumindest in einer Richtung, der Dosierrichtung, unabhängig vom ersten Begrenzungselement begrenzt. Die Antriebseinrichtung ist durch die Kupplung K2 lösbar drehfest mit der Dosiereinrichtung koppelbar, was es ermöglicht, das Antriebselement in Form der Spiralfeder 310 zu spannen.

Eine vierte Einheit umfasst die Kupplungshülse 120 und die Gewindestange 180, die eine starre Einheit bilden, die Elemente, auf denen diese Teile gelagert sind, nämlich die Führungshülse 110, den Lagerhalter 130 und die Kugellagerringe 140, sowie die Vorschubhülse 90. Diese Einheit stellt eine Fördereinrichtung dar, die eine Drehbewegung eines Eingangsglieds in Form der Kupplungshülse 120 in einen Vorschub des Förderelements in Form der Vorschubhülse 90 umwandelt. Ihr Eingangsglied ist durch die Kupplung K1 lösbar drehfest mit der Antriebseinrichtung koppelbar. Zudem ist es über die Kupplung K3 lösbar drehfest mit der Halteeinrichtung (d.h. dem Gehäuse) koppelbar.

Des weiteren ist eine Auslöseeinrichtung vorhanden, die primär den Druckknopf 80 umfasst und zur Bedienung der Kupplungen K1 bis K3 dient.

### Funktionsweise

Das Injektionsgerät wird wie folgt bedient. Ausgehend von der Ausgangsstellung der Fig. 16 wird zunächst eine zu verabreichende Dosis eingestellt. Hierzu wird die Dosierhülse 60 im Uhrzeigersinn gedreht. Die Dosierhülse nimmt dabei über die Kupplung K2 die Kupplungsscheibe 270 und die Kupplungswelle 230 mit. Hierdurch wird die Spiralfeder 310 aufgezogen. Das hierdurch entstehende Drehmoment wird durch die Ratschenverbindung zwischen dem mitdrehenden Ratschenring 260 und der stationären Anschlaghülse 240 gehalten. Durch die Drehung der Kupplungswelle 230 werden auch die Übertragungshülse 210 und die darauf geführte Anzeigetrommel 70 mitgedreht. Die Anzeigetrommel 70 wird wegen ihrer Gewindeführung am Mechanikhalter 150 zudem axial in proximaler Richtung verschoben, führt also insgesamt eine Schraubenbewegung in proximaler Richtung aus. Hierdurch wandern Markierungen auf der Oberfläche der Anzeigetrommel 70 unter dem Fenster 21 durch und zeigen die aktuell eingestellte Dosis an. Des weiteren wird der Dosisbegrenzungsring 220 aufgrund seines Gewindeeingriffs mit dem Inneren der Übertragungshülse 210 und aufgrund seiner drehgesicherten Anordnung auf der Kupplungshülse 120 in proximaler Richtung verschoben.

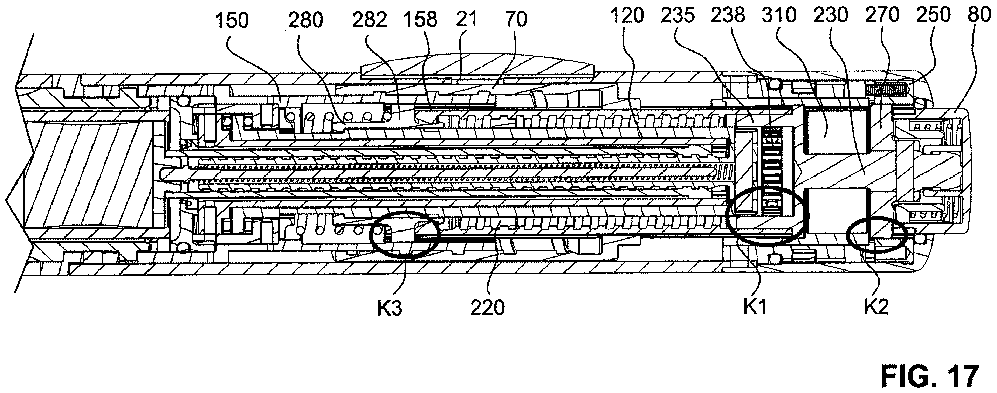

Die Fig. 17 zeigt das Injektionsgerät, nachdem die halbe maximale Einzeldosis eingestellt wurde. Die Anzeigetrommel ist den halben Weg zwischen ihrer distalen und ihrer proximalen Endstellung nach hinten gewandert. Ausserdem ist der Dosisbegrenzungsring 220 um einen Betrag in die proximale Richtung gewandert, der proportional zur eingestellten Einzeldosis ist.

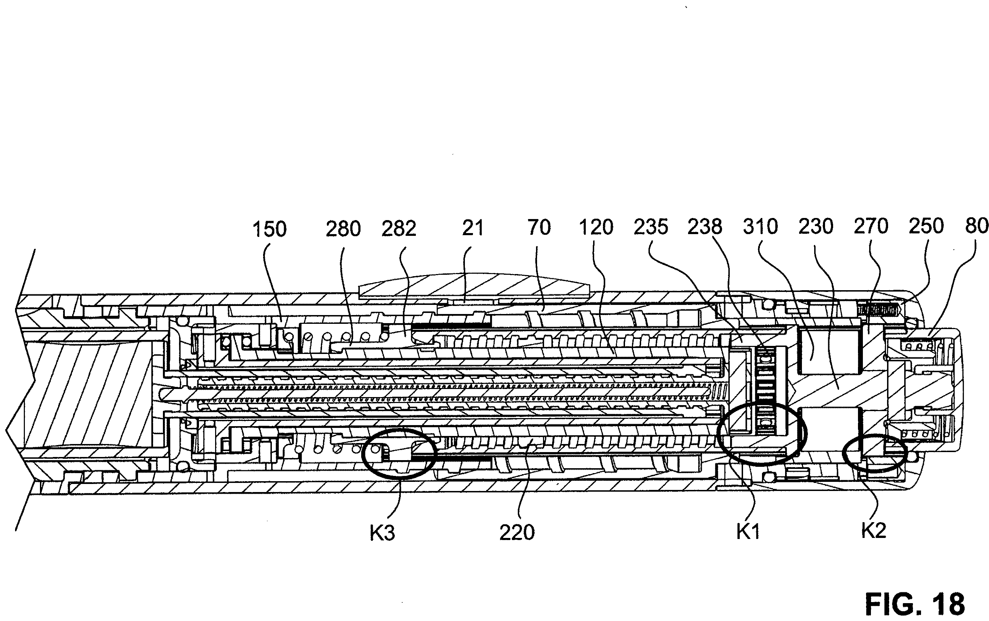

Die Drehung der Dosierhülse 60 im Uhrzeigersinn wird einerseits durch den maximalen Bewegungsbereich der Anzeigetrommel 70, andererseits durch den maximalen Bewegungsbereich des Dosisbegrenzungsrings 220 limitiert. Die Anzeigetrommel 70 stösst nach einer vorbestimmten Zahl von Umdrehungen der Dosierhülse 60 mit ihrem proximalen Radialanschlag gegen die Anschlaghülse 240, sofern die Drehung der Dosierhülse 60 nicht schon zuvor durch den Dosisbegrenzungsring 220 limitiert wurde, wie unten näher beschrieben wird. Dadurch ist kein Weiterdrehen der Dosierhülse 60 mehr möglich. Diese Stellung entspricht der maximal einstellbaren Einzeldosis. Diese Situation ist in der Fig. 18 dargestellt.

Soll die eingestellte Dosis korrigiert, also verringert, werden, so lässt sich die Dosierhülse 60 gegen die Kraft der Ratschenverbindung im Gegenuhrzeigersinn zurückdrehen. Da die Ratschenverbindung in diese Richtung zusätzlich auch das Drehmoment der Spiralfeder 310 aufzunehmen hat, ist die Ratschenverbindung asymmetrisch ausgestaltet: Die stirnseitige Verzahnung weist auf derjenigen Seite, die durch ein Drehmoment belastet ist, das im Gegenuhrzeigersinn auf die Dosierhülse wirkt, einen grösseren Steigungswinkel auf als auf derjenigen Seite, die bei einem Drehmoment im Uhrzeigersinn belastet wird (vgl. die Ausgestaltung der Zähne 244 in Fig. 14). Unter dem Steigungswinkel wird dabei der Absolutbetrag des Winkels zwischen der jeweiligen Flanke eines Zahnes auf der Stirnseite des Ratschenrings 260 bzw. auf der Stirnseite der Anschlaghülse 240 und einer Querschnittsfläche durch den Injektor verstanden.

Die Ausschüttung der so eingestellten Dosis erfolgt, indem der Druckknopf 80 eingedrückt wird. Hierdurch wird zunächst die Kupplung K1 eingekuppelt. Dadurch entsteht eine drehfeste Verbindung zwischen der Kupplungswelle 230 einerseits und der Kupplungshülse 120 sowie der damit starr verbundenen Gewindestange 180 andererseits. Nun sind alle drei Kupplungen K1, K2 und K3 eingekuppelt. Beim weiteren Eindrücken des Druckknopfs 80 kuppelt die Kupplung K2 aus. Dadurch wird die drehfeste Verbindung zwischen der Dosierhülse 60 einerseits und der Kupplungswelle 230 mit der nun angekoppelten Kupplungshülse 120 und Gewindestange 180 andererseits aufgehoben. Dies führt dazu, dass die Ratschenverbindung das Drehmoment der Spiralfeder 310 nicht mehr aufnimmt. Das System ist aber immer noch drehfest über die Kupplung K3 im Mechanikhalter 150 und damit in der Gehäusehülse 20 gehalten. Wenn der Druckknopf 80 nun noch weiter eingedrückt wird, kuppelt auch die Kupplung K3 aus. In diesem Moment wird das Drehmoment der Spiralfeder 310 frei und wirkt über die Kupplungswelle 230 und die Kupplungshülse 120 auf die Gewindestange 190. Hierdurch werden diese Teile in eine Drehung im Gegenuhrzeigersinn versetzt. Durch ihren Gewindeeingriff mit der Gewindestange 190 erfährt die Vorschubhülse 90 eine axiale Verschiebung in distaler Richtung. Über den Vorschubflansch 100 schiebt die Vorschubhülse den Stopfen 41 in der Karpule 40 vor. Auf diese Weise wird das Medikament ausgeschüttet.

Während der Ausschüttung wirken erhebliche Axialkräfte auf die Vorschubhülse 90: Das Drehmoment der Spiralfeder 310 wird in eine Kraft in Vorschubrichtung umgewandelt, die den Stopfen 41 in der Karpule 40 vortreibt. Diese Kräfte werden durch die Kugellager zwischen der Kupplungshülse 120 einerseits und der Führungshülse 110 und dem Lagerhalter 130 andererseits reibungsarm aufgenommen, so dass reibungsbedingte Gegenkräfte, die das antreibende Drehmoment mindern könnten, minimiert werden.

Bei der Ausschüttung wird die Anzeigetrommel 70 durch die Drehung der Übertragungshülse 210 im Gegenuhrzeigersinn wiederum mitgenommen und dadurch wegen ihres Gewindeeingriffs mit dem stationären Mechanikhalter 150 wieder in distaler Richtung bewegt, bis sie wieder ihre distale Ausgangsstellung einnimmt. In dieser Stellung wird sie durch einen Radialanschlag am Weiterdrehen gehindert, wodurch die Ausschüttung beendet wird. Nach Ende der Ausschüttung zeigt die Anzeigetrommel also wieder die Dosis "Null" an.

Die Ausschüttung kann jederzeit unterbrochen werden, indem der Druckknopf 80 losgelassen wird. Dadurch kuppeln die Kupplungen K3 und K2 wieder ein, und die Kupplung K1 kuppelt wieder aus. Die Anzeigetrommel 70 zeigt die verbleibende Restdosis an, die noch ausgeschüttet wird, wenn der Druckknopf wieder gedrückt wird und dadurch die Ausschüttung fortgesetzt wird.

Der Dosisbegrenzungsring 220 behält während der Ausschüttung seine axiale Lage bei, da die Übertragungshülse 210 und die Kupplungshülse 120, zwischen denen sich der Dosisbegrenzungsring 220 befindet, synchron drehen.

Nach dem Ende der Ausschüttung steht die Injektionsvorrichtung für den nächsten Injektionsvorgang bereit. Gegenüber der Fig. 16 haben jedoch zwei Bauteile ihre Position verändert: Einerseits ist die Vorschubhülse 90 entsprechend der ausgeschütteten Dosis in die distale Richtung gewandert. Andererseits ist der Dosisbegrenzungsring 220 ebenfalls um einen dazu proportionalen Betrag in proximaler Richtung gewandert. Hiervon abgesehen, entspricht der Zustand nach dem Ende der Injektion dem Ausgangszustand der Fig. 16. Mit jeder weiteren Injektion wandert also die Vorschubhülse 90 weiter in die distale Richtung, während der Dosisbegrenzungsring 220 in die proximale Richtung wandert. Dies ist in der Fig. 19 illustriert, welche die Injektionsvorrichtung darstellt, nachdem zunächst eine erste Dosis verabreicht wurde, die der halben maximalen Einzeldosis entspricht, und anschliessend mit der Dosierhülse erneut eine Dosis eingestellt wurde, die wiederum der halben maximalen Einzeldosis entspricht. Die Anzeigetrommel zeigt nun erneut, wie in der Fig. 15, die halbe maximale Einzeldosis an, während der Dosisbegrenzungsring 220 nun eine Position in der Übertragungshülse 210 einnimmt, die der Summe der bisher eingestellten Dosen, hier also zweimal der halben maximalen Einzeldosis, entspricht.

Der maximale axiale Weg, um den der Dosisbegrenzungsring 220 in proximaler Richtung in der Übertragungshülse wandern kann, entspricht gerade dem Inhalt einer vollständig gefüllten Karpule. Sobald die Summe der eingestellten Dosen an der Dosierhülse dem Karpuleninhalt entspricht, erreicht der Dosisbegrenzungsring 220 seine proximale Endstellung und stösst mit seinem Radialanschlag am axialen Ringflansch 235 der Kupplungswelle 230 an, wie dies in der Fig. 12A dargestellt ist. Dadurch wird die Dosierhülse 60 an einem weiteren Verdrehen im Uhrzeigersinn gehindert. Es kann also keine grössere Dosis eingestellt werden als diejenige Dosis, die der verbleibenden Restmenge des Medikaments in der Karpule entspricht. Die Fig. 20 zeigt diese Situation, in welcher kein Aufdosieren mehr möglich ist, obwohl sich die Anzeigetrommel in der distalen Ausgangsstellung, also der Nullstellung, befindet. Entsprechend hat die Vorschubhülse 90 ihre distale Endstellung erreicht, ist also maximal ausgefahren.

### Karpulenwechsel

Um die Karpule auszuwechseln, wird die Karpulenhülse 30 gegen den elastischen Widerstand der Bajonettfeder 170 aus dem Mechanikhalter 150 gelöst und durch den entsprechenden Führungsschlitz 156 im Mechanikhalter geführt herausgedreht. Hierdurch wird zwangsweise die Bajonetthülse 160 entlang ihrem eigenen, parallelen Führungsschlitz 155 ebenfalls verdreht und gleichzeitig in distaler Richtung verschoben. Hierdurch wird die Führungshülse 110 in die distale Richtung gezogen. Hierdurch wandern auch alle damit axial verbundenen beweglichen Teile, insbesondere die Kupplungshülse 120, die Gewindestange 190, die Arretierhülse 280, die Übertragungshülse 210, die Kupplungswelle 230, die Kupplungsscheibe 270 und der Druckknopf 80 in die distale Richtung. Insbesondere wird der Druckknopf 80 also in die Dosierhülse 60 eingezogen und zeigt so an, dass das Injektionsgerät nicht betriebsbereit ist.

Durch diese axiale Verschiebung der verschiedenen Teile der Mechanik kommen die Kupplungen K2 und K3 ausser Eingriff, während K1 ohnehin schon ausser Eingriff war. Sollte vor dem Karpulenwechsel noch eine Dosis eingestellt, aber nicht verabreicht worden sein, so versetzt die dementsprechend aufgezogene Spiralfeder 310 nun die Kupplungswelle 230 und die damit verbundene Übertragungshülse 210 in eine Drehung im Gegenuhrzeigersinn, bis die Anzeigetrommel 70 ihre distale Endstellung erreicht hat und durch ihren Radialanschlag am Mechanikhalter 150 ein weiteres Zurückdrehen verhindert. Auf diese Weise wird die Anzeigetrommel 70 in ihre distale Ausgangsstellung, die Nullstellung, zurückgebracht. Es erfolgt also eine automatische Rückstellung der Dosisanzeige auf Null.

Falls sich vor dem Karpulenwechsel noch eine Restmenge des Medikaments in der Karpule 40 befunden haben sollte, so war die Vorschubhülse 90 vor dem Karpulenwechsel noch nicht maximal ausgefahren, hatte also noch nicht ihre distale Endstellung erreicht. Beim Abnehmen der Karpulenhülse 30 drückt die Schraubenfeder 190 nun die Führungsnadel 200, den Vorschubflansch 100 und die Vorschubhülse 90 in die distale Richtung. Hierdurch wird die Gewindestange 180 über ihre Schraubenverbindung mit dem Inneren der Vorschubhülse 90 in Drehung versetzt. Dabei nimmt die Gewindestange 180 insbesondere die Kupplungshülse 120 und den Dosisbegrenzungsring 220 mit. Der Dosisbegrenzungsring 220 wird bei dieser Drehung durch seinen Gewindeeingriff mit der Übertragungshülse 210 in seine proximale Endstellung verschoben. Sobald der Dosisbegrenzungsring 220 diese Ausgangsstellung erreicht hat, verhindert er eine weitere Drehung der Kupplungshülse 120 und der Gewindestange 180, so dass kein weiteres Ausfahren der Vorschubhülse 90 mehr möglich ist. Die Vorschubhülse 90 hat dadurch nun ihre distale Endstellung erreicht. Diese Situation ist in der Fig. 1 dargestellt. Insgesamt befindet sich nun also die Anzeigetrommel 70 in der Nullstellung, der Dosisbegrenzungsring 220 in der proximalen Endstellung und die Vorschubhülse 90 in ihrer distalen Endstellung.

Nun wird eine neue Karpule 40 in die Karpulenhülse 30 eingeschoben, und die Karpulenhülse 30 mit der darin aufgenommenen Karpule 40 wird axial in die proximale Richtung gegen die Gehäusehülse 20 geführt. Dabei drückt zunächst der Stopfen 41 der Karpule den Vorschubflansch 100 und die Vorschubhülse 90 gegen die Kraft der Schraubenfeder 190 in die proximale Richtung. Hierdurch wird wiederum die Gewindestange 180 in Drehung versetzt. Die Gewindestange nimmt erneut die Kupplungshülse 120 und den Dosisbegrenzungsring 220 mit. Dadurch wird der Dosisbegrenzungsring aufgrund seines Gewindeeingriffs mit der Übertragungshülse 210 in die distale Richtung, d.h. in Richtung seiner Ausgangsstellung verschoben. Der Betrag, um den er in diese Richtung verschoben wird, entspricht gerade der in der Karpule 40 vorhandenen Dosis. Bei einer vollständig gefüllten Karpule wandert der Dosisbegrenzungsring 220 in seine distale Ausgangsstellung. Die Karpulenhülse 30 wird dann in den Mechanikhalter 150 eingeschoben, wobei die radialen Zapfen 36 der Karpulenhülse 30 wieder in die Führungsschlitze 156 im Mechanikhalter 150 eingreifen (vgl. Figuren 1 und 3). Durch die Zwangsführung der Karpulenhülse 30 beim Einsetzen in den Mechanikhalter 150 wird wiederum die Bajonetthülse 160 gezwungen, der Bewegung der Karpulenhülse 30 in den entsprechenden Führungsschlitzen zu folgen. Die Bajonetthülse 160 wird dadurch zurück in ihre proximale Endstellung gebracht, in welcher sie durch die Bajonettscheibe 170 lösbar verriegelt wird (vgl. Figuren 8 bis 10). Das Injektionsgerät befindet sich nun wieder in der Ausgangsstellung der Fig. 16 und steht nach dem Aufschrauben eines neuen Nadelhalters 31 für eine neue Abfolge von Verabreichungen zur Verfügung.

### Zweite Ausführungsform

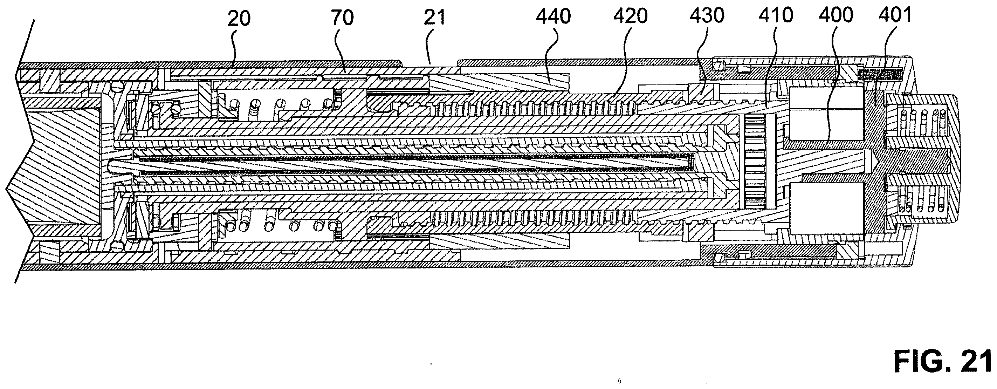

In der Fig. 21 ist als Variante eine zweite Ausführungsform des erfindungsgemässen Injektionsgeräts dargestellt. Die Funktionsweise ist im Wesentlichen die selbe wie bei der ersten Ausführungsform. Gleich wirkende Teile sind daher mit den gleichen Bezugsziffern bezeichnet wie in der ersten Ausführungsform. Im Folgenden soll lediglich auf die wesentlichen Unterschiede eingegangen werden.

In der zweiten Ausführungsform entfällt die Anschlaghülse 240. Ihre Funktion wird von der entsprechend verlängerten Gehäusehülse 20 übernommen.

Die Antriebseinrichtung, die in der ersten Ausführungsform, abgesehen von der Spiralfeder 310, aus der Kupplungsscheibe 270, Kupplungswelle 230 und Übertragungshülse 210 gebildet wird, ist hier durch andere Teile gebildet, nämlich durch eine Verbindungswelle 400 (mit daran einstückig ausgebildeter Kupplungsscheibe 401), eine am proximalen Ende geschlossene erste Übertragungshülse 410 und eine daran distal anschliessende zweite Übertragungshülse 420. Diese drei Teile sind wiederum starr miteinander verbunden.

Während in der ersten Ausführungsform die Anzeigetrommel nicht nur zur Anzeige der eingestellten Dosis diente, sondern auch zur Begrenzung der maximal einstellbaren Einzeldosis in Dosierrichtung und zur Begrenzung der Bewegung in Ausschüttrichtung, wird die letztere Funktion in der zweiten Ausführungsform durch einen zweiten Dosisbegrenzungsring 430 übernommen. Dieser ist drehfest, aber axial verschiebbar in der Gehäusehülse 20 geführt. Mit einem Innengewinde läuft er auf einem entsprechenden Aussengewinde der ersten Übertragungshülse 410. Seine axiale Bewegung ist durch zwei Radialanschläge zwischen einer distalen Ausgangsstellung, welcher der Nullstellung entspricht, und einer proximalen Endstellung, welcher der maximal einstellbaren Dosis entspricht, begrenzt. Auf diese Weise übernimmt er die Anschlagsfunktionen der Anzeigetrommel gemäss der ersten Ausführungsform.

Die Anzeigetrommel 70 ist in der zweiten Ausführungsform über eine starr mit ihr verbundene Mitnahmehülse 440 axial verschiebbar und drehfest auf der zweiten Übertragungshülse 420 geführt. Ihre Funktionsweise ist ansonsten gleich wie bei der ersten Ausführungsform.

Von diesen Unterschieden abgesehen, sind Aufbau und Funktionsweise des Injektionsgeräts im Wesentlichen gleich wie bei der ersten Ausführungsform.

### Weitere Varianten

Die Unterschiede zwischen der ersten und der zweiten Ausführungsform zeigen, dass die Funktionen des erfindungsgemässen Injektionsgeräts auf vielfältige Weise erreicht werden können und die Erfindung in keiner Weise auf die oben dargestellten Ausführungsbeispiele beschränkt ist. Verschiedene weitere Abwandlungen sind möglich. Diese können insbesondere durch fertigungstechnische Anforderungen bedingt sein.

### Bezugszeichenliste

- R: Medikamentenreservoir
- D1: Dichtung
- D2: Dichtung
- D3: Dichtung
- D4: Dichtung
- K1: erste Kupplung
- K2: zweite Kupplung
- K3: dritte Kupplung
- 10: Schutzhülse
- 11: Haltering
- 12: Rastarm
- 20: Gehäusehülse
- 21: Fenster
- 22: Fensterabdeckung
- 30: Karpulenhülse
- 31: Nadelhalter
- 32: Hohlnadel
- 33: Nadelschutzhülle
- 34: Sichtfenster
- 35: Verriegelungsbereich
- 36: Zapfen
- 40: Karpule
- 41: Stopfen
- 50: Schutzkappe
- 60: Dosierhülse
- 61: Federring
- 70: Anzeigetrommel
- 72: ringförmiger Bereich
- 73: Radialanschlag
- 80: Druckknopf
- 81: Arm
- 82: Schraubenfeder
- 83: Auflagering
- 90: Vorschubhülse
- 91: Führungsnocken
- 92: Innengewinde
- 100: Vorschubflansch
- 110: Führungshülse
- 111: Flansch
- 112: Bohrung
- 120: Kupplungshülse
- 121: Hülsenkörper
- 122: Längsrippe
- 123: Verdickung
- 124: Flansch
- 130: Lagerhalter
- 132: Schulter
- 140: Kugellagerring
- 141: Lagerkugel
- 150: Mechanikhalter
- 151: distaler Abschnitt
- 152: proximaler Abschnitt
- 153: Stufe
- 154: Längsschlitz
- 155: Führungsschlitz
- 156: Führungsschlitz
- 157: Aussengewinde
- 158: Längsnut
- 159: Radialanschlag
- 160: Bajonetthülse
- 161: Ringflansch
- 162: Arm
- 163: Öffnung
- 170: Bajonettfeder
- 171: Grundkörper
- 172: Vorsprung
- 173: Zunge
- 180: Aussengewindestange
- 181: Querbolzen
- 190: Schraubenfeder
- 200: Führungsnadel
- 201: Verdickung
- 202: Zapfen
- 210: Übertragungshülse
- 211: Innengewinde
- 212: Längsrippe
- 213: Ringflansch
- 220: Dosisbegrenzungsring
- 221: Aussengewinde
- 222: Längsnut
- 223: Radialanschlag
- 230: Kupplungswelle
- 231: Achse
- 232: Querbohrung
- 233: proximales Ende
- 234: Flansch
- 235: Ringflansch
- 236: Radialanschlag
- 237: Bohrung
- 238: Längsnut
- 240: Anschlaghülse
- 242: Loch
- 243: Radialanschlag
- 244: Zahn
- 250: Innenring
- 251: Bohrung
- 252: Schraubenfeder
- 260: Ratschenring
- 270: Kupplungsscheibe
- 271: Stift
- 280: Arretierhülse
- 281: Hauptkörper
- 282: Arm
- 283: Flanschbereich
- 284: Längsnut
- 290: Kupplungsfeder
- 300: Stützring
- 310: Spiralfeder
- 311: Federhaltebereich
- 320: Federhülse
- 400: Verbindungswelle
- 401: Kupplungsscheibe
- 410: erste Übertragungshülse
- 420: zweite Übertragungshülse
- 430: zweiter Dosisbegrenzungsring
- 440: Mitnahmehülse

## Patentansprüche

1. Injektionsgerät zur Injektion einer einstellbaren Dosis eines fluiden Produkts umfassend:
ein Gehäuse (20),
eine Antriebseinrichtung mit einer Vorschubhülse (90) und einer Gewindestange (180),
wobei die Vorschubhülse (90) ein Innengewinde (92) aufweist; das mit einem Aussengewinde der Gewindestange (180) in Eingriff steht, wobei die Gewindestange (180) relativ zum Gehäuse (20) fixierbar und in eine Drehbewegung versetzbar ist zum Fördern des Produkts aus einer Karpule (40),
eine Übertragungshülse (210), welche ein Innengewinde (211) aufweist,
eine Kupplungshülse (120),
einen Dosisbegrenzungsring (220) der die Einstellung einer Dosis verhindert die grösser ist als die in der Karpule (40) verbleibende Restmenge des Produkts,
wobei der Dosisbegrenzungsring sich zwischen der Übertragungshülse (210) und der Kupplungshülse (120) befindet,
**dadurch gekennzeichnet, dass**
der Dosisbegrenzungsring (220) ein Außengewinde (221) aufweist, das in das Innengewinde (211) der Übertragungshülse (210) eingreift und in Bezug zur Kupplungshülse (120) drehfest und axial verschiebbar ist, wobei der Dosisbegrenzungsring (220) während der Dosiseinstellung in proximale oder distale Richtung verschoben werden kann proportional zur eingestellten Dosis.

2. Injektionsgerät nach Anspruch 1, **dadurch gekennzeichnet, dass** der Dosisbegrenzungsring (220) einen Radialanschlag aufweist, der in seiner Endstellung an einer Gegenfläche anstößt, sobald die Summe der eingestellten Dosen dem Inhalt der Karpule (40) entspricht.

3. Injektionsgerät nach Anspruch 2, **dadurch gekennzeichnet, dass** der Dosisbegrenzungsring (220) in seiner proximalen oder distalen Endstellung an einer Gegenfläche anstößt.

4. Injektionsgerät nach einem der vorgehenden Ansprüche, **dadurch gekennzeichnet, dass** der Dosisbegrenzungsring (220 an der Kupplungshülse (120) drehfest und axial verschiebbar geführt ist.

5. Injektionsgerät nach einem der vorhergehenden Ansprüche, **dadurch gekennzeichnet, dass** der maximale axiale Weg, um den der Dosisbegrenzungsring (220) in proximaler oder distaler_Richtung bewegbar ist, dem Inhalt einer vollständig gefüllten Karpule (40) entspricht.

6. Injektionsgerät nach einem der vorhergehenden Ansprüche, **dadurch gekennzeichnet, dass** die Kupplungshülse (120) und die Übertragungshülse (210) während der Dosiseinstellung relativ zueinander verdrehbar sind und sich während der Dosisausschüttung synchron drehen.

7. Injektionsgerät nach einem der vorhergehenden Ansprüche, wobei die Antriebseinrichtung angepasst ist, eine Drehbewegung der Kupplungshülse (120) in einen Vorschub der Vorschubhülse (90) umzuwandeln.

8. Injektionsgerät nach Anspruch 7, **dadurch gekennzeichnet, dass** die Kupplungshülse (120) über die Gewindestange (180) mit der Vorschubhülse (90) verbunden ist.

9. Injektionsgerät nach Anspruch 8, **dadurch gekennzeichnet, dass** die Vorschubhülse (90) in Bezug auf das Gehäuse (20) drehfest und axial verschiebbar ist und/oder auf einen Stopfen (41) der Karpule (40) wirkt.

10. Injektionsgerät nach Anspruch 9, **dadurch gekennzeichnet, dass** die Vorschubhülse (90) an einer zum Gehäuse (20) drehfest angeordneten Führungshülse (110) drehfest und in Längsrichtung verschiebbar gelagert ist und nur im Bereich seines proximalen Endes längsgeführt ist.

11. Injektionsgerät nach Anspruch 10, **dadurch gekennzeichnet, dass** die Vorschubhülse (90) relativ zum Gehäuse (20) entlang einer Vorschubachse über einen Vorschubbereich zwischen einer proximalen und distalen Endstellung bewegbar ist.

12. Injektionsgerät nach Anspruch 11, **dadurch gekennzeichnet, dass** die Führungshülse (110) einer Länge hat die mindestens dem Vorschubbereich des Förderelements (90) entspricht.

13. Injektionsgerät nach Anspruch 12, **dadurch gekennzeichnet, dass** die Gewindestange (180) im Inneren der Vorschubhülse (90) angeordnet ist und sich zumindest über eine Länge von proximal nach distal in das Förderelement (90) hinein erstreckt die dem Vorschubbereich entspricht.

14. Injektionsgerät nach Anspruch 12 oder 13 **dadurch gekennzeichnet, dass** die Vorschubhülse (90) auf seiner Aussenseite im Wesentlichen glatt ausgestaltet ist

## Claims

1. Injection device for injecting an adjustable dose of a fluid product, comprising:
a housing (20),
a drive means comprising a feed sleeve (90) and a threaded rod (180), the feed sleeve (90) having an internal thread (92) which engages with an external thread of the threaded rod (180), the threaded rod (180) being fixable relative to the housing (20) and settable into a rotary motion for conveying the product from a cartridge (40),
a transmission sleeve (210) which has an internal thread (211),
a coupling sleeve (120),
a dose limiting ring (220) which prevents the setting of a dose greater than the residual amount of product remaining in the cartridge (40),
the dose limiting ring being located between the transmission sleeve (210) and the coupling sleeve (120),
**characterized in that**
the dose limiting ring (220) has an external thread (221) that engages in the internal thread (211) of the transmission sleeve (210) and is rotationally fixed and axially displaceable with respect to the coupling sleeve (120), the dose limiting ring (220) being displaceable in a proximal or distal direction during dose setting proportional to the set dose.

2. Injection device according to claim 1, **characterized in that** the dose limiting ring (220) has a radial stop which, in its end position, abuts a counter surface as soon as the sum of the set doses corresponds to the contents of the cartridge (40).

3. Injection device according to claim 2, **characterized in that** the dose limiting ring (220) abuts a counter surface in its proximal or distal end position.

4. Injection device according to any of the preceding claims,
**characterized in that** the dose limiting ring (220) is guided in a rotationally fixed and axially displaceable manner on the coupling sleeve (120).

5. Injection device according to any of the preceding claims,
**characterized in that** the maximum axial distance by which the dose limiting ring (220) can be moved in a proximal or distal direction corresponds to the contents of a fully filled cartridge (40).

6. Injection device according to any of the preceding claims,
**characterized in that** the coupling sleeve (120) and the transmission sleeve (210) during dose setting are rotatable relative to each other and rotate synchronously during dose delivery.

7. Injection device according to any of the preceding claims, wherein the drive means is adapted to convert a rotary movement of the coupling sleeve (120) into a feed of the feed sleeve (90).

8. Injection device according to claim 7, **characterized in that** the coupling sleeve (120) is connected to the feed sleeve (90) via the threaded rod (180).

9. Injection device according to claim 8, **characterized in that** the feed sleeve (90) is rotationally fixed and axially displaceable with respect to the housing (20) and/or acts on a plug (41) of the cartridge (40).

10. Injection device according to claim 9, **characterized in that** the feed sleeve (90) is mounted on a guide sleeve (110) in a rotationally fixed manner and displaceable in the longitudinal direction, the guide sleeve being arranged in a rotationally fixed manner relative to the housing (20), and the feed sleeve is guided longitudinally only in the region of its proximal end.

11. Injection device according to claim 10, **characterized in that** the feed sleeve (90) is movable relative to the housing (20) along a feed axis over a feed range between a proximal and distal end position.

12. Injection device according to claim 11, **characterized in that** the guide sleeve (110) has a length that corresponds at least to the feed range of the conveying element (90).

13. Injection device according to claim 12, **characterized in that** the threaded rod (180) is arranged inside the feed sleeve (90) and extends at least over a length from proximal to distal into the conveying element (90) which length corresponds to the feed range.

14. Injection device according to claim 12 or 13 **characterized in that** the feed sleeve (90) is essentially smooth on its outer surface

## Revendications

1. Appareil d'injection permettant d'injecter une dose réglable d'un produit fluide comprenant :
un boîtier (20),
un dispositif d'entraînement comportant un manchon d'avance (90) et une tige filetée (180), dans lequel le manchon d'avance (90) présente un filetage interne (92) qui est en prise avec un filetage externe de la tige filetée (180), dans lequel la tige filetée (180) peut être fixée par rapport au boîtier (20) et peut être décalée dans un mouvement de rotation pour transporter le produit hors d'une carpule (40),
un manchon de transmission (210), qui présente un filetage interne (211),
un manchon de couplage (120),
une bague de limitation de dose (220) qui empêche le réglage d'une dose qui est supérieure à la quantité résiduelle de produit restant dans la carpule (40),
dans lequel la bague de limitation de dose se trouve entre le manchon de transmission (210) et le manchon de couplage (120),
**caractérisé en ce que**
la bague de limitation de dose (220) présente un filetage externe (221) qui vient en prise dans le filetage interne (211) du manchon de transmission (210) et est fixe en rotation et déplaçable axialement par rapport au manchon de couplage (120), dans lequel la bague de limitation de dose (220) peut être déplacée pendant le réglage de la dose dans la direction proximale ou distale proportionnellement à la dose réglée.

2. Appareil d'injection selon la revendication 1, **caractérisé en ce que** la bague de limitation de dose (220) présente une butée radiale qui, dans sa position finale, vient buter contre une contre-surface dès que la somme des doses réglées correspond au contenu de la carpule (40).

3. Appareil d'injection selon la revendication 2, **caractérisé en ce que** la bague de limitation de dose (220) dans sa position finale proximale ou distale vient buter contre une contre-surface.

4. Appareil d'injection selon l'une des revendications précédentes, **caractérisé en ce que** la bague de limitation de dose (220 est guidée sur le manchon de couplage (120) de manière fixe en rotation et déplaçable axialement.

5. Appareil d'injection selon l'une des revendications précédentes, **caractérisé en ce que** la course axiale maximale sur laquelle la bague de limitation de dose (220) est mobile dans la direction proximale ou distale correspond au contenu d'une carpule (40) entièrement remplie.

6. Appareil d'injection selon l'une des revendications précédentes, **caractérisé en ce que** le manchon de couplage (120) et le manchon de transmission (210) sont rotatifs l'un par rapport à l'autre pendant le réglage de dose et entrent en rotation de manière synchrone pendant la distribution de dose.

7. Appareil d'injection selon l'une des revendications précédentes, dans lequel le dispositif d'entraînement est adapté pour convertir un mouvement de rotation du manchon de couplage (120) en une avance du manchon d'avance (90).

8. Appareil d'injection selon la revendication 7, **caractérisé en ce que** le manchon de couplage (120) est relié au manchon d'avance (90) par l'intermédiaire de la tige filetée (180).

9. Appareil d'injection selon la revendication 8, **caractérisé en ce que** le manchon d'avance (90) est fixe en rotation et déplaçable axialement par rapport au boîtier (20) et/ou agit sur un bouchon (41) de la carpule (40).

10. Appareil d'injection selon la revendication 9, **caractérisé en ce que** le manchon d'avance (90) est monté sur un manchon de guidage (110) agencé fixe en rotation par rapport au boîtier (20), de manière fixe en rotation et déplaçable dans la direction longitudinale, et n'est guidé longitudinalement que dans une zone de son extrémité proximale.

11. Appareil d'injection selon la revendication 10, **caractérisé en ce que** le manchon d'avance (90) est mobile par rapport au boîtier (20) le long d'un axe d'avance sur une plage d'avance entre une position finale proximale et une position finale distale.

12. Appareil d'injection selon la revendication 11, **caractérisé en ce que** le manchon de guidage (110) présente une longueur qui correspond au moins à la plage d'avance de l'élément de transport (90).

13. Dispositif d'injection selon la revendication 12, **caractérisé en ce que** la tige filetée (180) est agencée à l'intérieur du manchon d'avance (90) et s'étend au moins sur une longueur du côté proximal au côté distal dans l'élément de transport (90) qui correspond à la zone d'avance.

14. Appareil d'injection selon la revendication 12 ou 13,
**caractérisé en ce que** le manchon d'avance (90) est conçu de manière essentiellement lisse sur son côté externe
